# EUROPEAN PATENT APPLICATION

(11) **EP 4 800 024 A1**
(43) Date of publication of application: **02.09.2026**
(21) Application number: 25161006.9
(22) Date of filing: 28.02.2025
(51) Int. Cl.: C07K 7/08, C07K 14/005, A61K 38/00

(54) **TRANSDUCTION-ENHANCING PEPTIDES AND USES THEREOF**

(71) Applicant: JPT Peptide Technologies GmbH, 12489 Berlin (DE)
(72) Inventor: SCHNATBAUM, Karsten, 12489 Berlin (DE)
(74) Representative: Maiwald GmbH

(57) **Abstract**

The present invention relates to peptides that have been found to influence infection of viruses and transduction efficiency of viruses, viral vectors or virus-like particles, respectively, into target cells as well as compositions containing said peptides, methods of use thereof, and cells obtained by using said peptides for their transduction.

## Description

### Field of the Invention

The present invention relates to peptides that have been found to influence infection of viruses and transduction efficiency of viruses, viral vectors or virus-like particles, respectively, into target cells as well as compositions containing said peptides, methods of use thereof, and cells obtained by using said peptides for their transduction.

### Background

Gene therapy requires effective transfer of genetic information to target cells. To date, many approaches use transduction by viruses, e.g. retroviruses, for this purpose. However, existing technologies suffer from various drawbacks, one of which are low transduction efficacies.

While there exist various possibilities to increase transduction efficacy including the use of various compounds that promote viral gene delivery, they typically suffer from at least one of the following disadvantages: low practicability (e.g. requirement to coat reaction vessels), low solubility, fibril formation, cytotoxicity, and low transduction enhancement.

In view of these difficulties, recently peptides promoting transduction efficacy have been proposed. Peptides are particularly interesting for this purpose, since they are generally biodegradable, have reduced size compared to proteins or polymers, and are relatively simple to characterize and produce. Known peptides that have been proposed for this purpose include fibronectin, natural cationic peptides derived from HIV1, LAH4 peptides, as for example described in EP2452947 A1, WO 2013/001041 A1, and WO 2014/177635 A1.

While the peptides described in the art provide a useful toolbox, many of the known peptides still suffer from low transduction efficacy, cytotoxicity, and low solubility.

There is thus need in the art for peptides that overcome at least part of the disadvantages of existing peptides.

### Summary of the Invention

The inventors of the present invention have surprisingly identified novel transduction enhancing peptides that provide, compared to previously described transduction enhancing peptides, increased retroviral transduction efficacy, higher aqueous solubility, reduced or absent fibril formation, and/or reduced cytotoxicity.

In a first aspect, the invention therefore relates to a peptide comprising the amino acid sequence:
X¹X²X³X⁴X⁵X⁶X⁷X⁸X⁹X¹⁰X¹¹X¹²
wherein
X¹ is absent or is any amino acid, preferably pyroglutamic acid (Pyr), A, D, E, F, G, H, I, K, L, M, N, P, Q, R, S, T, V, W, Y, or ornithine (Orn), more preferably pyroglutamic acid (Pyr), H, K, R, P, T, Q or ornithine (Orn), optionally comprising an N-terminal protecting group, preferably selected from C₁-C₁₀-alkyl-C(=O)-, C₆-C₁₄-aryl-C(=O)-, C₁-C₁₀-alkyl-NH-C(=O)-, C₆-C₁₄-aryl-NH-C(=O)-; (C₁-C₁₀-alkyl)₂N-C(=O)-, (C₆-C₁₄-aryl)₂N-C(=O)-, C₁-C₁₀-alkyl, C₁-C₁₀-alkyl-O-C(=O)-, C₆-C₁₄-aryl-O-C(=O)-, C₁-C₁₀-alkyl-S(O)₂-, C₆-C₁₄-aryl-S(O)₂-, more preferably acetyl, propyl, phenyl, methyl-NH-C(=O), phenyl-NH-C(=O)-, methyl-O-C(=O)-, phenyl-O-C(=O)-, methyl-S(O)₂-, phenyl-S(O)₂-, CH₃, and (CH₃)₂;
X² is absent or S, diaminopropionic acid (Dpr), aminobutyric acid (Abu), H, G, A, P, N, L-homoserine (Hse), or C;
X³ is K or R;
X⁴ is I, L, V, F, W, Y, M, or K;
X⁵ is K or R;
X⁶ is Q, K, I, R, T, or S;
X⁷ is I, L or V;
X⁸ is S, F, A or M;
X⁹ is I or V;
X¹⁰ is L-norleucine (Nle), L-norvaline (Nva), F, Y, M, L, I, Q, T, 2-aminoheptanoic acid (Gly(C5)), 2-aminooctanoic acid (Gly(C6)), or W;
X¹¹ is W, F, I, or L;
X¹² is Q, H, R, S or K.

In various embodiments of the peptide
(1) X¹ is Pyr, H, K, R, P, T, Q or Orn, preferably Pyr, H, P or R;
(2) X² is S, Dpr, Abu, H, G, A, P, N or C, preferably S, Dpr, H, G, A, or C;
(3) X³ is K or R;
(4) X⁴ is I, L or V, preferably I;
(5) X⁵ is K;
(6) X⁶ is Q or K, preferably Q;
(7) X⁷ is I;
(8) X⁸ is S or F, preferably S;
(9) X⁹ is I;
(10) X¹⁰ is Nle or F, preferably Nle;
(11) X¹¹ is W; and/or
(12) X¹² is Q, H, R or S, preferably Q, R or H.

In various embodiments, the peptide comprises the amino acid sequence:
(1)

   X¹X²X³X⁴KX⁶IX⁸IX¹⁰WX¹²;
(2)

   X¹X²X³IKX⁶IX⁸IX¹⁰WX¹²;
(3)

   X¹X²X³LKX⁶IX⁸IX¹⁰WX¹²;
(4)

   X¹X²X³VKX⁶IX⁸IX¹⁰WX¹²;
(5)

   X¹X²X³X⁴KQIX⁸IX¹⁰WX¹²;
(6)

   X¹X²X³X⁴KKIX⁸IX¹⁰WX¹²;
(7)

   X¹X²X³X⁴KIIX⁸IX¹⁰WX¹²
(8)

   X¹X²X³X⁴KX⁶ISIX¹⁰WX¹²;
(9)

   X¹X²X³X⁴KX⁶IFIX¹⁰WX¹²;
(10)

   X¹X²X³X⁴KX⁶IX⁸I-Nle-WX¹²;
(11)

   X¹X²X³X⁴KX⁶IX⁸IX¹⁰WQ;
(12)

   X¹X²X³X⁴KX⁶IX⁸IX¹⁰WR;
(13)

   X¹X²X³IKQISIX¹⁰X¹¹X¹²; or
(14)

   X¹X²X³IKQISIX¹⁰WX¹².

In various embodiments, the peptide comprises the amino acid sequence:
(1)

   X¹X²KX⁴KX⁶IX⁸IX¹⁰WX¹²;
(2)

   X¹X²KIKX⁶IX⁸IX¹⁰WX¹²;
(3)

   X¹X²KLKX⁶IX⁸IX¹⁰WX¹²;
(4)

   X¹X²KVKX⁶IX⁸IX¹⁰WX¹²;
(5)

   X¹X²KX⁴KQIX⁸IX¹⁰WX¹²;
(6)

   X¹X²KX⁴KKIX⁸IX¹⁰WX¹²;
(7)

   X¹X²KX⁴KIIX⁸IX¹⁰WX¹²
(8)

   X¹X²KX⁴KX⁶ISIX¹⁰WX¹²;
(9)

   X¹X²KX⁴KX⁶IFIX¹⁰WX¹²;
(10)

   X¹X²KX⁴KX⁶IX⁸I-Nle-WX¹²;
(11)

   X¹X²KX⁴KX⁶IX⁸IX¹⁰WQ;
(12)

   X¹X²KX⁴KX⁶IX⁸IX¹⁰WR;
(13)

   X¹X²KX⁴KX⁶IX⁸IX¹⁰WH;
(14)

   X¹X²KIKQISIX¹⁰X¹¹X¹²; or
(15)

   X¹X²KIKQISIX¹⁰WX¹².

In various embodiments, the peptide comprises the amino acid sequence:

X¹X²KIKQISIX¹⁰WX¹²

wherein X¹⁰ is preferably Nle and/or X¹² is preferably Q. In various embodiments, the peptide thus comprises the amino acid sequence X¹X²KIKQISI-Nle-WQ.

In various embodiments, the peptide comprises or consists of the amino acid sequence:
(1) Pyr-SKLKQISI-Nle-WQ (SEQ ID NO:1);
(2) PSKIKQISI-Nle-WQ (SEQ ID NO:2);
(3) HHKIKQISI-Nle-WQ (SEQ ID NO:3);
(4) RSRIKQISI-Nle-WQ (SEQ ID NO:4), wherein the N-terminus is acetylated;
(5) HSKIKQISI-Nle-WQ (SEQ ID NO:5);
(6) Pyr-Hse-KIKQISI-Nle-WQ (SEQ ID NO:6);
(7) Pyr-Dpr-KIKQISI-Nle-WQ (SEQ ID NO:7);
(8) H-Dpr-KIKQISI-Nle-WQ (SEQ ID NO:8);
(9) R-Dpr-KIKQISI-Nle-WQ (SEQ ID NO:9);
(10) KSKIKQISI-Nle-WQ (SEQ ID NO:10), wherein the N-terminus is acetylated;
(11) RSKIKQISI-Nle-WQ (SEQ ID NO:11), wherein the N-terminus is acetylated;
(12) Pyr-SKIKKISI-Nle-WQ (SEQ ID NO:12);
(13) Pyr-SKIKQISI-Nle-WR (SEQ ID NO:13);
(14) Orn-SKIKQISI-Nle-WQ (SEQ ID NO:14), wherein the N-terminus is acetylated;
(15) RSKIKQISI-Nle-WQ (SEQ ID NO:15);
(16) HCKIKQISI-Nle-WQ (SEQ ID NO:16);
(17) RHKIKQISI-Nle-WQ (SEQ ID NO:17);
(18) RCKIKQISI-Nle-WQ (SEQ ID NO:18);
(19) RSRIKQISI-Nle-WQ (SEQ ID NO:19);
(20) Pyr-SKIKQISI-Nle-WQ (SEQ ID NO:20);
(21) ASKIKQISI-Nle-WQ (SEQ ID NO:21), wherein the N-terminus is acetylated;
(22) GSKIKQISI-Nle-WQ (SEQ ID NO:22), wherein the N-terminus is acetylated;
(23) HSKIKQISI-Nle-WQ (SEQ ID NO:23), wherein the N-terminus is acetylated;
(24) NSKIKQISI-Nle-WQ (SEQ ID NO:24), wherein the N-terminus is acetylated;
(25) PSKIKQISI-Nle-WQ (SEQ ID NO:25), wherein the N-terminus is acetylated;
(26) QSKIKQISI-Nle-WQ (SEQ ID NO:26), wherein the N-terminus is acetylated;
(27) SSKIKQISI-Nle-WQ (SEQ ID NO:27), wherein the N-terminus is acetylated;
(28) TSKIKQISI-Nle-WQ (SEQ ID NO:28), wherein the N-terminus is acetylated;
(29) Pyr-AKIKQISI-Nle-WQ (SEQ ID NO:29);
(30) Pyr-GKIKQISI-Nle-WQ (SEQ ID NO:30);
(31) Pyr-HKIKQISI-Nle-WQ (SEQ ID NO:31);
(32) Pyr-NKIKQISI-Nle-WQ (SEQ ID NO:32);
(33) Pyr-PKIKQISI-Nle-WQ (SEQ ID NO:33);
(34) Pyr-SRIKQISI-Nle-WQ (SEQ ID NO:34);
(35) Pyr-SKFKQISI-Nle-WQ (SEQ ID NO:35);
(36) Pyr-SKKKQISI-Nle-WQ (SEQ ID NO:36);
(37) Pyr-SKVKQISI-Nle-WQ (SEQ ID NO:37);
(38) Pyr-SKWKQISI-Nle-WQ (SEQ ID NO:38);
(39) Pyr-SKIRQISI-Nle-WQ (SEQ ID NO:39);
(40) Pyr-SKIKRISI-Nle-WQ (SEQ ID NO:40);
(41) Pyr-SKIKSISI-Nle-WQ (SEQ ID NO:41);
(42) Pyr-SKIKQIMI-Nle-WQ (SEQ ID NO:42);
(43) Pyr-SKIKQISIFWQ (SEQ ID NO:43);
(44) Pyr-SKIKQISIIWQ (SEQ ID NO:44);
(45) Pyr-SKIKQISILWQ (SEQ ID NO:45);
(46) Pyr-SKIKQISIMWQ (SEQ ID NO:46);
(47) Pyr-SKIKQISIQWQ (SEQ ID NO:47);
(48) Pyr-SKIKQISITWQ (SEQ ID NO:48);
(49) Pyr-SKIKQISIWWQ (SEQ ID NO:49);
(50) Pyr-SKIKQISIYWQ (SEQ ID NO:50);
(51) Pyr-SKIKQISI-Nle-FQ (SEQ ID NO:51);
(52) Pyr-SKIKQISI-Nle-WH (SEQ ID NO:52);
(53) Pyr-SKIKQISI-Nle-WK (SEQ ID NO:53);
(54) Pyr-SKIKQISI-Nle-WS (SEQ ID NO:54);
(55) KGGQSKIKQISI-Nle-WQ (SEQ ID NO:55), wherein the N-terminus is acetylated;
(56) KGQSKIKQISI-Nle-WQ (SEQ ID NO:56), wherein the N-terminus is acetylated;
(57) KQSKIKQISI-Nle-WQ (SEQ ID NO:57), wherein the N-terminus is acetylated;
(58) KISKIKQISI-Nle-WQ (SEQ ID NO:58), wherein the N-terminus is acetylated;
(59) HHQSKIKQISI-Nle-WQ (SEQ ID NO:59), wherein the N-terminus is acetylated;
(60) Pyr-SKIKQISI-Nle-WQHH (SEQ ID NO:60);
(61) SKIKQISI-Nle-WQ (SEQ ID NO:61), wherein the N-terminus is acetylated;
(62) Pyr-KIKQISI-Nle-WQ (SEQ ID NO:62);
(63) ASKIKQISI-Nle-WQ (SEQ ID NO:63);
(64) DSKIKQISI-Nle-WQ (SEQ ID NO:64);
(65) ESKIKQISI-Nle-WQ (SEQ ID NO:65);
(66) FSKIKQISI-Nle-WQ (SEQ ID NO:66);
(67) GSKIKQISI-Nle-WQ (SEQ ID NO:67);
(68) ISKIKQISI-Nle-WQ (SEQ ID NO:68);
(69) KSKIKQISI-Nle-WQ (SEQ ID NO:69);
(70) LSKIKQISI-Nle-WQ (SEQ ID NO:70);
(71) MSKIKQISI-Nle-WQ (SEQ ID NO:71);
(72) NSKIKQISI-Nle-WQ (SEQ ID NO:72);
(73) QSKIKQISI-Nle-WQ (SEQ ID NO:73);
(74) SSKIKQISI-Nle-WQ (SEQ ID NO:74);
(75) TSKIKQISI-Nle-WQ (SEQ ID NO:75);
(76) VSKIKQISI-Nle-WQ (SEQ ID NO:76);
(77) WSKIKQISI-Nle-WQ (SEQ ID NO:77);
(78) YSKIKQISI-Nle-WQ (SEQ ID NO:78);
(79) Pyr-Abu-KIKQISI-Nle-WQ (SEQ ID NO:79);
(80) Pyr-AKIKQISI-Nle-WQ (SEQ ID NO:80);
(81) Pyr-SKIKQISI-Nva-WQ (SEQ ID NO:81);
(82) Pyr-SKIKQISI-Gly(C5)-WQ (SEQ ID NO:82);
(83) Pyr-SKIKQISI-Gly(C6)-WQ (SEQ ID NO:83);
(84) Me2Q-SKIKQISI-Nle-WQ (SEQ ID NO:84);
(85) Dpr-bAla-QSKIKQISI-Nle-WQ (SEQ ID NO:85); or
(86) Pyr-CKIKQISI-Nle-WQ (SEQ ID NO:86).

In such embodiments, the peptide may preferably comprise or consist of the amino acid sequence
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4), wherein the N-terminus is acetylated;
KSKIKQISI-Nle-WQ (SEQ ID NO:10), wherein the N-terminus is acetylated;
RSKIKQISI-Nle-WQ (SEQ ID NO:11), wherein the N-terminus is acetylated;
Pyr-SKIKKISI-Nle-WQ (SEQ ID NO:12);
Pyr-SKIKQISI-Nle-WR (SEQ ID NO:13);
Orn-SKIKQISI-Nle-WQ (SEQ ID NO:14), wherein the N-terminus is acetylated;
HSKIKQISI-Nle-WQ (SEQ ID NO:5);
RSKIKQISI-Nle-WQ (SEQ ID NO:15);
Pyr-Hse-KIKQISI-Nle-WQ (SEQ ID NO:6);
Pyr-Dpr-KIKQISI-Nle-WQ (SEQ ID NO:7);
H-Dpr-KIKQISI-Nle-WQ (SEQ ID NO:8);
HCKIKQISI-Nle-WQ (SEQ ID NO:16);
RHKIKQISI-Nle-WQ (SEQ ID NO:17);
R-Dpr-KIKQISI-Nle-WQ (SEQ ID NO:9);
RCKIKQISI-Nle-WQ (SEQ ID NO:18);
RSRIKQISI-Nle-WQ (SEQ ID NO:19);
HSKIKQISI-Nle-WQ (SEQ NO:23), wherein the N-terminus is acetylated;
PSKIKQISI-Nle-WQ (SEQ NO:25), wherein the N-terminus is acetylated;
Pyr-AKIKQISI-Nle-WQ (SEQ NO:29);
Pyr-GKIKQISI-Nle-WQ (SEQ NO:30);
Pyr-HKIKQISI-Nle-WQ (SEQ NO:31);
Pyr-SKVKQISI-Nle-WQ (SEQ NO:37);
KQSKIKQISI-Nle-WQ (SEQ NO:57), wherein the N-terminus is acetylated;
Pyr-KIKQISI-Nle-WQ (SEQ NO:62);
ASKIKQISI-Nle-WQ (SEQ NO:63);
LSKIKQISI-Nle-WQ (SEQ NO:70);
MSKIKQISI-Nle-WQ (SEQ NO:71);
QSKIKQISI-Nle-WQ (SEQ NO:73);
SSKIKQISI-Nle-WQ (SEQ NO:74);
YSKIKQISI-Nle-WQ (SEQ NO:78);
Pyr-Abu-KIKQISI-Nle-WQ (SEQ NO:79);
Pyr-AKIKQISI-Nle-WQ (SEQ NO:80); or
Pyr-CKIKQISI-Nle-WQ (SEQ NO:86);
preferably any one of
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4), wherein the N-terminus is acetylated.

In various embodiments, the peptide is 10 to 30 amino acids in length, preferably 10 to 20 amino acids in length, more preferably 11 to 15 amino acids in length.

In various embodiments, the peptide has the ability to improve the transduction efficiency of a virus or viral vector relative to the same virus or viral vector without the presence of the peptide.

In another aspect, the invention is directed to a composition comprising at least one peptide as described herein. The composition may further comprise at least one virus or viral vector.

In one aspect of the invention, the compositions described herein are for use in transducing a eukaryotic cell.

In still another aspect, the compositions described herein are for use as a medicament or biopharmaceutical.

In a further aspect, the compositions described herein are for use in gene therapy.

The present invention further relates, in another aspect, to a method for the infection of a eukaryotic cell by a virus or viral vector, comprising contacting the cell with the virus or viral vector in the presence of at least one peptide as described herein.

In a further aspect, the present invention is also directed to a eukaryotic cell transduced with a composition of the invention or obtained by the methods of the invention described herein.

Another aspect of the invention relates to a method for increasing the efficiency of nucleic acid transfer into a eukaryotic target cell with a viral vector, comprising contacting the target cell with the viral vector in the presence of at least one peptide according to the invention.

In still another aspect, the invention relates to use of at least one peptide of the invention for promoting the infection of a eukaryotic cell by a virus or viral vector.

The invention is also directed, in another aspect, to use of at least one peptide of the invention in a method of diagnosis, for example for diagnosing a disease or disorder.

In the compositions for use, the methods, the eukaryotic cells or the uses of the invention, the eukaryotic cell may be selected from immune cells, preferably lymphocytes and/or the virus may be selected from retroviruses.

### Detailed Description

The terms used herein have, unless explicitly stated otherwise, the meanings as commonly understood in the art.

"At least one", as used herein, relates to one or more, in particular 1, 2, 3, 4, 5, 6, 7, 8, 9, 10 or more. "At least", if used in relation to a numerical value and in particular a list of numerical values, relates to each of said separate numerical values and defines said numerical value as the minimum value. "At least one", if used in relation to a non-numerical species or molecule, typically refers to the respective type of molecule or species but not the number of molecules. "At least one peptide" thus typically means at least one type of peptide but not at least one peptide molecule, unless the context suggests otherwise.

The term "peptide" is used throughout the specification to designate a polymer of amino acid residues connected to each other by peptide bonds. A peptide according to the present invention includes dipeptides and oligopeptides and may have 2-100 amino acid residues. The terms "protein" and "polypeptide" are used interchangeably throughout the specification to designate a polymer of amino acid residues connected to each other by peptide bonds. A protein or polypeptide according to the present invention has preferably more than 100 amino acid residues. The peptides of the invention can comprise any one of the 20 natural proteinogenic amino acids, i.e. any one of glycine (G), alanine (A), valine (V), leucine (L), isoleucine (I), phenylalanine (F), tyrosine (Y), tryptophan (W), serine (S), threonine (T), cysteine (C), methionine (M), proline (P), asparagine (N), glutamine (Q), glutamic acid €, aspartic acid (D), histidine (H), lysine (K), and arginine (R). In addition to these, the peptides may comprise one or more further amino acids, which may be natural or non-natural amino acids. These include, without limitation, pyroglutamic acid (Pyr), ornithine (Orn), norleucine (Nle), diaminopropionic acid (Dpr), aminobutyric acid (Abu), homoserine (Hse), 2-aminoheptanoic acid and 2-aminooctanoic acid. All amino acids referred to herein are typically the L-form of the amino acids, unless explicitly indicated differently. In the peptides of the invention, the amino acids are linked by common peptide bonds, unless indicated differently. All peptide sequences referred to herein are shown in N- to C-terminal orientation from left to right. For all standard amino acids, herein the one letter code is used for designation, as given above.

"Flanking sequence", as used herein, relates to an amino acid sequence that is in the immediate vicinity of a given sequence element, i.e. is connected to the N- or C-terminus of said given sequence element by a peptide bond. The flanking sequence may have a length as defined herein, with the positional numbering of such a flanking sequence starting such that position 1 is the amino acid directly neighboring the sequence element and then counting away from the sequence element that is flanked either upstream, i.e. in the direction of the N-terminus if it flanks the N-terminus of the sequence element, or in direction of the C-terminus if it flanks the C-terminus of the sequence element, unless explicitly indicated otherwise.

The present invention is based on the inventors' identification of certain peptides that have increased transduction efficacy for viruses and viral vectors and therefore may have utility in various fields, including therapeutic applications, such as gene therapy, and in cell biology and cell culture. The peptides have further been found to provide not only for high transduction efficacy but also to have reduced cytotoxicity good stability and/or good solubility, in particular relative to other peptides known for a similar purpose.

The peptides of the invention comprise the amino acid sequence:

X¹X²X³X⁴X⁵X⁶X⁷X⁸X⁹X¹⁰X¹¹X¹²

wherein
X¹ is absent or is any amino acid, preferably pyroglutamic acid (Pyr), A, D, E, **F,** G, H, I, K, L, M, N, P, Q, R, S, T, V, W, Y, or ornithine (Orn), more preferably pyroglutamic acid (Pyr), H, K, R, P, T, Q or ornithine (Orn), optionally comprising an N-terminal protecting group, preferably selected from C₁-C₁₀-alkyl-C(=O)-, C₆-C₁₄-aryl-C(=O)-, C₁-C₁₀-alkyl-NH-C(=O)-, C₆-C₁₄-aryl-NH-C(=O)-; (C₁-C₁₀-alkyl)₂N-C(=O)-, (C₆-C₁₄-aryl)₂N-C(=O)-, C₁-C₁₀-alkyl, C₁-C₁₀-alkyl-O-C(=O)-, C₆-C₁₄-aryl-O-C(=O)-, C₁-C₁₀-alkyl-S(O)₂-, C₆-C₁₄-aryl-S(O)₂-, more preferably acetyl, propyl, phenyl, methyl-NH-C(=O), phenyl-NH-C(=O)-, methyl-O-C(=O)-, phenyl-O-C(=O)-, methyl-S(O)₂-, phenyl-S(O)₂-, CH₃, and (CH₃)₂;
X² is absent or is S, diaminopropionic acid (Dpr), aminobutyric acid (Abu), H, G, A, P, N, homoserine (Hse), or C;
X³ is K or R;
X⁴ is I, L, V, F, W, Y, M, or K;
X⁵ is K or R;
X⁶ is Q, K, I, R, T, or S;
X⁷ is I, L or V;
X⁸ is S, F, A or M;
X⁹ is I or V;
X¹⁰ is L-norleucine (Nle), L-norvaline (Nva), F, Y, M, L, I, Q, T, 2-aminoheptanoic acid, 2-aminooctanoic acid, or W;
X¹¹ is W, **F,** I, or L;
X¹² is Q, H, R, S or K.

The peptide of the invention is at least a decapeptide, i.e. 10 amino acids in length, since X¹ and X² may both be absent but X³ to X¹² are always present.

Pyroglutamic acid (Pyr) is also known as 5-oxoproline, pidolic acid, or 2-pyrrolidone-5-carboxylic acid and has the following structure

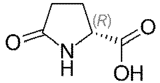

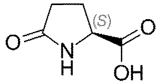

The S enantiomer corresponds to the L form of said amino acid and is the preferred enantiomer in the peptides of the invention.

Ornithine (Orn) is 2,5-diaminovaleric acid and is preferably L-ornithine. It has the following structure (L-form)

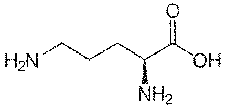

Diaminopropionic acid (Dpr) is 2,3-diaminopropanoic acid or 2,3-diaminopropionate and is also known as 3-Amino-(L)-alanine. Its L-form, which is the preferred enantiomer, has the following structure

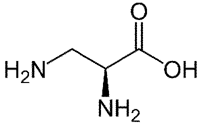

Aminobutyric acid (Abu), as used herein, refers to alpha-aminobutyric acid (2-aminobutanoic acid), also known as homoalanine and has the following structure 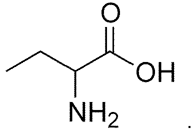 The L-form, i.e. L-2-aminobutyric acid, is the preferred enantiomer in the peptides of the invention

Homoserine (Hse) is also called isothreonine and is 2-amino-4-hydroxylbutanoic acid and has the following structure (L-homoserine) 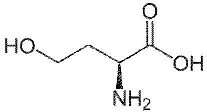 The L-form, i.e. L-homoserine, is the preferred enantiomer in the peptides of the invention.

Norleucin (Nle) is chemically 2-aminohexanoic acid and has the following structure (L-Form) 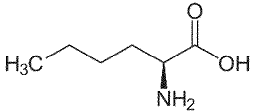 The L-form, i.e. L-norleucine, is the preferred enantiomer in the peptides of the invention.

Norvaline (Nva) is chemically 2-aminopentanoic acid and has the following structure (L-form) 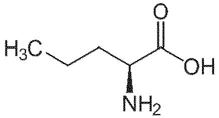 The L-form, i.e. L-norvaline, is the preferred enantiomer in the peptides of the invention.

Dimethyl-L-glutamine (Me2Q), chemically (2S)-N2-dimethyl-2,5-diamino-5-oxopentanoic acid, may also be present in the peptides of the invention as indicated herein below. It has the following structure (L-form):

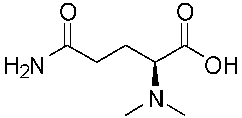

Beta-alanine (bAla) is chemically 3-aminopropanoic acid and has the following structure:

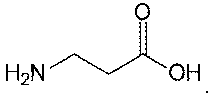

2-Aminoheptanoic acid (abbreviated herein "Gly(C5)") has the structure 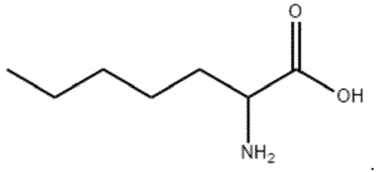 The L-form thereof is the preferred enantiomer used in the peptides of the invention. In all embodiments disclosed herein that include 2-aminoheptanoic acid, the amino acid is preferably L-2-aminoheptanoic acid.

2-Aminooctanoic acid (abbreviated herein "Gly(C6)") is also known as 2-Aminocaprylic acid and has the structure 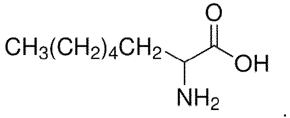 The L-form thereof is the preferred enantiomer used in the peptides of the invention. In all embodiments disclosed herein that include 2-aminooctanoic acid, the amino acid is preferably L-2-aminooctanoic acid.

The N-terminal protecting group is a group that replaces one or both of the hydrogen atoms of the terminal amino group. It is preferably selected from C₁-C₁₀-alkyl-C(=O)-, C₁-C₁₀-hydroxyalkyl-C(=O)-, C₆-C₁₄-aryl-C(=O)-, C₁-C₁₀-alkyl-NH-C(=O)-, C₆-C₁₄-aryl-NH-C(=O)-; (C₁-C₁₀-alkyl)₂N-C(=O)-, (C₆-C₁₄-aryl)₂N-C(=O)-, C₁-C₁₀-alkyl, (C₁-C₁₀-alkyl)₂-, C₁-C₁₀-alkyl-O-C(=O)-, C₆-C₁₄-aryl-O-C(=O)-, C₁-C₁₀-alkylS(O)₂-, C₆-C₁₄-aryl-S(O)₂-, more preferably acetyl, hydroxyacetyl, propionyl, benzoyl, methyl-NH-C(=O), phenyl-NH-C(=O)-, methyl-O-C(=O)-, phenyl-O-C(=O)-, methyl-S(O)₂-, phenyl-S(O)₂-, and CH₃. In various embodiments, only one hydrogen of the terminal amino group is replaced by one of the listed groups. In case the protecting group is methyl, the terminal amino group may be an N,N-dimethylamino group. The preferred protecting group is acetyl, i.e. CH₃-C(=O)-, so that the N-terminus is acetylated.

X¹ can be any amino acid, which includes A, C, D, E, F, G, H, I, K, L, M, N, P, Q, R, S, T, V, W, Y, and all other amino acids specifically disclosed herein, such as Pyr, Orn, Abu, Nle, Nla, Hse, Dpr, Me2Q, and bAla. In various embodiments, X¹ is pyroglutamic acid (Pyr), A, D, E, F, G, H, I, K, L, M, N, P, Q, R, S, T, V, W, Y, or ornithine (Orn), preferably Pyr, H, K, R, P, T, Q or Orn, more preferably Pyr, H, P or R.

In various embodiments, X² is S, Dpr, Abu, H, G, A, P, N or C, preferably S, Dpr, H, G or A.

In various embodiments, X³ is K or R.

In various embodiments, X⁴ is I, L or V, preferably I.

In various embodiments, X⁵ is K, i.e. the peptide comprises the sequence X¹X²X³X⁴KX⁶X⁷X⁸X⁹X¹⁰X¹¹X¹²
In various embodiments, X⁶ is Q or K, preferably Q.

In various embodiments, X⁷ is I, i.e. the peptide comprises the sequence X¹X²X³X⁴X⁵X⁶1X⁸X⁹X¹⁰X¹¹X¹²
In various embodiments, X⁸ is S or F, preferably S.

In various embodiments, X⁹ is I, i.e. the peptide comprises the sequence X¹X²X³X⁴X⁵X⁶X⁷X⁸¹X¹⁰X¹¹X¹²
In various embodiments, X¹⁰ is Nle or F, preferably Nle.

In various embodiments, X¹¹ is W, i.e. the peptide comprises the sequence X¹X²X³X⁴X⁵X⁶X⁷X⁸X⁹X¹⁰WX¹²
In various embodiments, X¹² is Q, H, R or S, preferably Q, R or H.

In various embodiments of the peptides of the invention
(1) X¹ is Pyr, H, K, R, P, T, Q or Orn, preferably Pyr, H, P or R;
(2) X² is S, Dpr, Abu, H, G, A, P, N or C, preferably S, Dpr, H, G or A;
(3) X³ is K or R;
(4) X⁴ is I, L or V, preferably I;
(5) X⁵ is K;
(6) X⁶ is Q or K, preferably Q;
(7) X⁷ is I;
(8) X⁸ is S or F, preferably S;
(9) X⁹ is I;
(10) X¹⁰ is Nle or F, preferably Nle;
(11) X¹¹ is W; and/or
(12) X¹² is Q, H, R or S, preferably Q, R or H.

In various embodiments, the definitions are at least combined as follows: (1) and (2), (1) and (3), (1) and (4), (1) and (5), (1) and (6), (1) and (7), (1) and (8), (1) and (9), (1) and (10), (1) and (11), (1) and (12), (2) and (3), (2) and (4), (2) and (5), (2) and (6), (2) and (7), (2) and (8), (2) and (9), (2) and (10), (2) and (11), (2) and (12), (3) and (4), (3) and (5), (3) and (6), (3) and (7), (3) and (8), (3) and (9), (3) and (10), (3) and (11), (3) and (12), (4) and (5), (4) and (6), (4) and (7), (4) and (8), (4) and (9), (4) and (10), (4) and (11), (4) and (12), (5) and (6), (5) and (7), (5) and (8), (5) and (9), (5) and (10), (5) and (11), (5) and (12), (6) and (7), (6) and (8), (6) and (9), (6) and (10), (6) and (11), (6) and (12), (7) and (8), (7) and (9), (7) and (10), (7) and (11), (7) and (12), (8) and (9), (8) and (10), (8) and (11), (8) and (12), (9) and (10), (9) and (11), (9) and (12), (10) and (11), (10) and (12), or (11) and (12).

In various embodiments, the definitions are at least combined as follows: (3), (4) and (5); (3), (4), and (6); (3), (4) and (7); (3), (4), and (8); (3), (4) and (9); (3), (4), and (10); (3), (4) and (11); (3), (4), and (12); (3), (5) and (6); (3), (5) and (7); (3), (5), and (8); (3), (5) and (9); (3), (5), and (10); (3), (5) and (11); (3), (5), and (12); (3), (6) and (7); (3), (6), and (8); (3), (6) and (9); (3), (6), and (10); (3), (6) and (11); (3), (6), and (12); (3), (7), and (8); (3), (7) and (9); (3), (7), and (10); (3), (7) and (11); (3), (7), and (12); (3), (8) and (9); (3), (8), and (10); (3), (8) and (11); (3), (8), and (12); (3), (9), and (10); (3), (9) and (11); (3), (9), and (12); (3), (10) and (11); (3), (10), and (12); (3), (11), and (12); (4), (5), and (6); (4), (5) and (7); (4), (5), and (8); (4), (5) and (9); (4), (5), and (10); (4), (5) and (11); (4), (5), and (12); (4), (6) and (7); (4), (6), and (8); (4), (6) and (9); (4), (6), and (10); (4), (6) and (11); (4), (6), and (12); (4), (7), and (8); (4), (7) and (9); (4), (7), and (10); (4), (7) and (11); (4), (7), and (12); (4), (8) and (9); (4), (8), and (10); (4), (8) and (11); (4), (8), and (12); (4), (9), and (10); (4), (9) and (11); (4), (9), and (12); (4), (10) and (11); (4), (10), and (12); (4), (11), and (12); (5), (6) and (7); (5), (6), and (8); (5), (6) and (9); (5), (6), and (10); (5), (6) and (11); (5), (6), and (12); (5), (7), and (8); (5), (7) and (9); (5), (7), and (10); (5), (7) and (11); (5), (7), and (12); (5), (8) and (9); (5), (8), and (10); (5), (8) and (11); (5), (8), and (12); (5), (9), and (10); (5), (9) and (11); (5), (9), and (12); (5), (10) and (11); (5), (10), and (12); (5), (11), and (12); (6), (7), and (8); (6), (7) and (9); (6), (7), and (10); (6), (7) and (11); (6), (7), and (12); (6), (8) and (9); (6), (8), and (10); (6), (8) and (11); (6), (8), and (12); (6), (9), and (10); (6), (9) and (11); (6), (9), and (12); (6), (10) and (11); (6), (10), and (12); (6), (11), and (12); (7), (8) and (9); (7), (8), and (10); (7), (8) and (11); (7), (8), and (12); (7), (9), and (10); (7), (9) and (11); (7), (9), and (12); (7), (10) and (11); (7), (10), and (12); (7), (11), and (12); (8), (9), and (10); (8), (9) and (11); (8), (9), and (12); (8), (10) and (11); (8), (10), and (12); (8), (11), and (12); (9), (10) and (11); (9), (10), and (12); (9), (11), and (12); or (10), (11), and (12).

In various embodiments, the definitions are at least combined as follows: (5), (7), (9) and (11); (5), (7), (9), (11) and any one of (3), (4), (6), (8), (10), and (12); (5), (7), (9), (11) and any two of (3), (4), (6), (8), (10), and (12); (5), (7), (9), (11) and any three of (3), (4), (6), (8), (10), and (12); (5), (7), (9), (11) and any four of (3), (4), (6), (8), (10), and (12); (5), (7), (9), (11) and any five of (3), (4), (6), (8), (10), and (12).

In various embodiments, the definitions are at least combined as follows: (3), (4), (5), (6), (7), (8), (9), (10) and (11) and optionally (12); (3), (4), (5), (6), (7), (8), (9), (10) and (11) and one of (1) and (2); (3), (4), (5), (6), (7), (8), (9), (10) and (11) and two of (1), (2), and (12).

In some embodiments, all definitions (1) to (12) are combined.

In various embodiments of the peptides of the invention
(1a) X¹ is Pyr, H, P or R;
(2a) X² is S, Dpr, H, G or A;
(3a) X³ is K or R;
(4a) X⁴ is I;
(5a) X⁵ is K;
(6a) X⁶ is Q;
(7a) X⁷ is I;
(8a) X⁸ is S;
(9a) X⁹ is I;
(10a) X¹⁰ is Nle;
(11a) X¹¹ is W; and/or
(12a) X¹² is Q, R or H.

In various embodiments, the definitions are at least combined as follows: (1a) and (2a), (1a) and (3a), (1a) and (4a), (1a) and (5a), (1a) and (6a), (1a) and (7a), (1a) and (8a), (1a) and (9a), (1a) and (10a), (1a) and (11a), (1a) and (12a), (2a) and (3a), (2a) and (4a), (2a) and (5a), (2a) and (6a), (2a) and (7a), (2a) and (8a), (2a) and (9a), (2a) and (10a), (2a) and (11a), (2a) and (12a), (3a) and (4a), (3a) and (5a), (3a) and (6a), (3a) and (7a), (3a) and (8a), (3a) and (9a), (3a) and (10a), (3a) and (11a), (3a) and (12a), (4a) and (5a), (4a) and (6a), (4a) and (7a), (4a) and (8a), (4a) and (9a), (4a) and (10a), (4a) and (11a), (4a) and (12a), (5a) and (6a), (5a) and (7a), (5a) and (8a), (5a) and (9a), (5a) and (10a), (5a) and (11a), (5a) and (12a), (6a) and (7a), (6a) and (8a), (6a) and (9a), (6a) and (10a), (6a) and (11a), (6a) and (12a), (7a) and (8a), (7a) and (9a), (7a) and (10a), (7a) and (11a), (7a) and (12a), (8a) and (9a), (8a) and (10a), (8a) and (11a), (8a) and (12a), (9a) and (10a), (9a) and (11a), (9a) and (12a), (10a) and (11a), (10a) and (12a), or (11a) and (12a).

In various embodiments, the definitions are at least combined as follows: (3a), (4a) and (5a); (3a), (4a), and (6a); (3a), (4a) and (7a); (3a), (4a), and (8a); (3a), (4a) and (9a); (3a), (4a), and (10a); (3a), (4a) and (11a); (3a), (4a), and (12a); (3a), (5a) and (6a); (3a), (5a) and (7a); (3a), (5a), and (8a); (3a), (5a) and (9a); (3a), (5a), and (10a); (3a), (5a) and (11a); (3a), (5a), and (12a); (3a), (6a) and (7a); (3a), (6a), and (8a); (3a), (6a) and (9a); (3a), (6a), and (10a); (3a), (6a) and (11a); (3a), (6a), and (12a); (3a), (7a), and (8a); (3a), (7a) and (9a); (3a), (7a), and (10a); (3a), (7a) and (11a); (3a), (7a), and (12a); (3a), (8a) and (9a); (3a), (8a), and (10a); (3a), (8a) and (11a); (3a), (8a), and (12a); (3a), (9a), and (10a); (3a), (9a) and (11a); (3a), (9a), and (12a); (3a), (10a) and (11a); (3a), (10a), and (12a); (3a), (11a), and (12a); (4a), (5a), and (6a); (4a), (5a) and (7a); (4a), (5a), and (8a); (4a), (5a) and (9a); (4a), (5a), and (10a); (4a), (5a) and (11a); (4a), (5a), and (12a); (4a), (6a) and (7a); (4a), (6a), and (8a); (4a), (6a) and (9a); (4a), (6a), and (10a); (4a), (6a) and (11a); (4a), (6a), and (12a); (4a), (7a), and (8a); (4a), (7a) and (9a); (4a), (7a), and (10a); (4a), (7a) and (11a); (4a), (7a), and (12a); (4a), (8a) and (9a); (4a), (8a), and (10a); (4a), (8a) and (11a); (4a), (8a), and (12a); (4a), (9a), and (10a); (4a), (9a) and (11a); (4a), (9a), and (12a); (4a), (10a) and (11a); (4a), (10a), and (12a); (4a), (11a), and (12a); (5a), (6a) and (7a); (5a), (6a), and (8a); (5a), (6a) and (9a); (5a), (6a), and (10a); (5a), (6a) and (11a); (5a), (6a), and (12a); (5a), (7a), and (8a); (5a), (7a) and (9a); (5a), (7a), and (10a); (5a), (7a) and (11a); (5a), (7a), and (12a); (5a), (8a) and (9a); (5a), (8a), and (10a); (5a), (8a) and (11a); (5a), (8a), and (12a); (5a), (9a), and (10a); (5a), (9a) and (11a); (5a), (9a), and (12a); (5a), (10a) and (11a); (5a), (10a), and (12a); (5a), (11a), and (12a); (6a), (7a), and (8a); (6a), (7a) and (9a); (6a), (7a), and (10a); (6a), (7a) and (11a); (6a), (7a), and (12a); (6a), (8a) and (9a); (6a), (8a), and (10a); (6a), (8a) and (11a); (6a), (8a), and (12a); (6a), (9a), and (10a); (6a), (9a) and (11a); (6a), (9a), and (12a); (6a), (10a) and (11a); (6a), (10a), and (12a); (6a), (11a), and (12a); (7a), (8a) and (9a); (7a), (8a), and (10a); (7a), (8a) and (11a); (7a), (8a), and (12a); (7a), (9a), and (10a); (7a), (9a) and (11a); (7a), (9a), and (12a); (7a), (10a) and (11a); (7a), (10a), and (12a); (7a), (11a), and (12a); (8a), (9a), and (10a); (8a), (9a) and (11a); (8a), (9a), and (12a); (8a), (10a) and (11a); (8a), (10a), and (12a); (8a), (11a), and (12a); (9a), (10a) and (11a); (9a), (10a), and (12a); (9a), (11a), and (12a); or (10a), (11a), and (12a).

In various embodiments, the definitions are at least combined as follows: (5a), (7a), (9a) and (11a); (5a), (7a), (9a), (11a) and any one of (3a), (4a), (6a), (8a), (10a), and (12a); (5a), (7a), (9a), (11a) and any two of (3a), (4a), (6a), (8a), (10a), and (12a); (5a), (7a), (9a), (11a) and any three of (3a), (4a), (6a), (8a), (10a), and (12a); (5a), (7a), (9a), (11a) and any four of (3a), (4a), (6a), (8a), (10a), and (12a); (5a), (7a), (9a), (11a) and any five of (3a), (4a), (6a), (8a), (10a), and (12a).

In various embodiments, the definitions are at least combined as follows: (3a), (4a), (5a), (6a), (7a), (8a), (9a), (10a) and (11a) and optionally (12a); (3a), (4a), (5a), (6a), (7a), (8a), (9a), (10a) and (11a) and one of (1a) and (2a); (3a), (4a), (5a), (6a), (7a), (8a), (9a), (10a) and (11a) and two of (1a), (2a), and (12a).

In some embodiments, all definitions (1a) to (12a) are combined.

In various embodiments, the peptide comprises any one of the following amino acid sequences:
(1)

   X¹X²X³X⁴KX⁶IX⁸IX¹⁰WX¹²;
(2)

   X¹X²X³IKX⁶IX⁸IX¹⁰WX¹²;
(3)

   X¹X²X³LKX⁶IX⁸IX¹⁰WX¹²;
(4)

   X¹X²X³VKX⁶IX⁸IX¹⁰WX¹²;
(5)

   X¹X²X³X⁴KQIX⁸IX¹⁰WX¹²;
(6)

   X¹X²X³X⁴KKIX⁸IX¹⁰WX¹²;
(7)

   X¹X²X³X⁴KIIX⁸IX¹⁰WX¹²
(8)

   X¹X²X³X⁴KX⁶ISIX¹⁰WX¹²;
(9)

   X¹X²X³X⁴KX⁶IFIX¹⁰WX¹²;
(10)

   X¹X²X³X⁴KX⁶IX⁸I-Nle-WX¹²;
(11)

   X¹X²X³X⁴KX⁶IX⁸IX¹⁰WQ;
(12)

   X¹X²X³X⁴KX⁶IX⁸IX¹⁰WR;
(13)

   X¹X²X³IKQISIX¹⁰X¹¹X¹²; or
(14)

   X¹X²X³IKQISIX¹⁰WX¹².

The peptide may thus comprise, consist essentially of or consist of any one of the amino acid sequences, X¹X²X³X⁴KX⁶IX⁸IX¹⁰WX¹²; X¹X²X³IKX⁶IX⁸IX¹⁰WX¹²; X¹X²X³LKX⁶IX⁸IX¹⁰WX¹²; X¹X²X³VKX⁶IX⁸IX¹⁰WX¹²; X¹X²X³X⁴KQIX⁸IX¹⁰WX¹²; X¹X²X³KKIX⁸IX¹⁰WX¹²; X¹X²X³X⁴KIIX⁸IX¹⁰WX¹²; X¹X²X³X⁴KX⁶ISIX¹⁰WX¹²; X¹X²X³X⁴KX⁶IFIX¹⁰WX¹²; X¹X²X³X⁴KX⁶IX⁸I-Nle-WX¹²; X¹X²X³X⁴KX⁶IX⁸IX¹⁰WQ; X¹X²X³X⁴KX⁶IX⁸IX¹⁰WR; X¹X²X³IKQISIX¹⁰X¹¹X¹²; or X¹X²X³IKQISIX¹⁰WX¹².

The peptide can comprise, consist essentially of, or consist of any one of the consensus sequences: X¹X²X³-(I/L/V)-KQIX⁸IX¹⁰WX¹²; X¹X²X³-(I/L/V)-KKIX⁸IX¹⁰WX¹²; X¹X²X³-(I/L/V)-KIIX⁸IX¹⁰WX¹²; X¹X²X³-(I/L/V)-KX⁶ISIX¹⁰WX¹²; X¹X²X³-(I/L/V)-KX⁶IFIX¹⁰WX¹²; X¹X²X³-(I/L/V)-KX⁶IX⁸I-Nle-WX¹²; X¹X²X³-(I/L/V)-KX⁶IX⁸IX¹⁰WQ; X¹X²X³-(I/L/V)-KX⁶IX⁸IX¹⁰WR; X¹X²X³X⁴K-(Q/K/I)-ISIX¹⁰WX¹²; X¹X²X³X⁴K-(Q/K/I)-IFIX¹⁰WX¹²; X¹X²X³X⁴K-(Q/K/I)-IX⁸I-Nle-WX¹²; X¹X²X³X⁴K-(Q/K/I)-IX⁸IX¹⁰WQ; X¹X²X³X⁴K-(Q/K/I)-IX⁸IX¹⁰WR; X¹X²X³X⁴KX⁶I-(S/F)-I-Nle-WX¹²; X¹X²X³X⁴KX⁶I-(S/F)-IX¹⁰WQ; X¹X²X³X⁴KX⁶I-(S/F)-IX¹⁰WR; X¹X²X³X⁴KX⁶IX⁸I-Nle-WQ; X¹X²X³X⁴KX⁶IX⁸I-Nle-WR; X¹X²X³-(I/L/V)-KQISIX¹⁰WX¹²; X¹X²X³-(I/L/V)-KQIFIX¹⁰WX¹²; X¹X²X³-(I/L/V)-KKISIX¹⁰WX¹²; X¹X²X³-(I/L/V)-KKIFIX¹⁰WX¹²; X¹X²X³-(I/L/V)-KIISIX¹⁰WX¹²; X¹X²X³-(I/L/V)-KIIFIX¹⁰WX¹²; X¹X²X³-(I/L/V)-KX⁶ISI-Nle-WX¹²; X¹X²X³-(I/L/V)-KX⁶IFI-Nle-WX¹²; X¹X²X³X⁴K-(Q/K/I)-ISIX¹⁰WQ; X¹X²X³X⁴K-(Q/K/I)-ISIX¹⁰WR; X¹X²X³X⁴K-(Q/K/I)-IFIX¹⁰WQ; X¹X²X³X⁴K-(Q/K/I)-IFIX¹⁰WR; X¹X²X³-(I/L/V)-KX⁶IX⁸I-Nle-WQ; X¹X²X³-(I/L/V)-KX⁶IX⁸I-Nle-WR; X¹X²X³X⁴K-(Q/K/I)-ISI-Nle-WX¹²; X¹X²X³X⁴K-(Q/K/I)-IFI-Nle-WX¹²; X¹X²X³X⁴K-(Q/K/I)-ISIX¹⁰WQ; X¹X²X³X⁴K-(Q/K/I)-ISIX¹⁰WR; X¹X²X³X⁴K-(Q/K/I)-IFIX¹⁰WQ; X¹X²X³X⁴K-(Q/K/I)-IFIX¹⁰WK; X¹X²X³X⁴K-(Q/K/I)-IX⁸I-Nle-WQ; X¹X²X³X⁴K-(Q/K/I)-IX⁸I-Nle-WR; X¹X²X³X⁴KX⁶I-(S/F)-I-Nle-WQ; X¹X²X³X⁴KX⁶I-(S/F)-I-Nle-WR; X¹X²X³-(I/L/V)-KQISI-Nle-WX¹²; X¹X²X³-(I/L/V)-KQIFI-Nle-WX¹²; X¹X²X³-(I/L/V)-KKISI-NIe-WX¹²; X¹X²X³-(I/L/V)-KKIFI-Nle-WX¹²; X¹X²X³-(I/L/V)-KIISI-Nle-WX¹²; X¹X²X³-(I/L/V)-KII FI-Nle-WX¹²; X¹X²X³-(I/L/V)-KQISIX¹⁰WQ; X¹X²X³-(I/L/V)-KQISIX¹⁰WR; X¹X²X³-(I/L/V)-KQIFIX¹⁰WQ; X¹X²X³-(I/L/V)-KQIFIX¹⁰WR; X¹X²X³-(I/L/V)-KKISIX¹⁰WQ; X¹X²X³-(I/L/V)-KKISIX¹⁰WR; X¹X²X³-(I/L/V)-KKIFIX¹⁰WQ; X¹X²X³-(I/L/V)-KKIFIX¹⁰WR; X¹X²X³-(I/L/V)-KIISIX¹⁰WQ; X¹X²X³-(I/L/V)-KIISIX¹⁰WR; X¹X²X³-(I/L/V)-KIIFIX¹⁰WQ; X¹X²X³-(I/L/V)-KIIFIX¹⁰WR; X¹X²X³-(I/L/V)-KX⁶ISI-Nle-WQ; X¹X²X³-(I/L/V)-KX⁶ISI-Nle-WR; X¹X²X³-(I/L/V)-KX⁶IFI-Nle-WQ; X¹X²X³-(I/L/V)-KX⁶IFI-Nle-WR; X¹X²X³X⁴K-(Q/K/I)-ISI-Nle-WQ; X¹X²X³X⁴K-(Q/K/I)-ISI-Nle-WR; X¹X²X³X⁴K-(Q/K/I)-IFI-Nle-WQ; X¹X²X³X⁴K-(Q/K/I)-IFI-Nle-WR; X¹X²X³-(I/L/V)-KQISI-Nle-WQ; X¹X²X³-(I/L/V)-KQISI-Nle-WR; X¹X²X³-(I/L/V)-KQIFI-Nle-WQ; X¹X²X³-(I/L/V)-KQIFI-Nle-WR; X¹X²X³-(I/L/V)-KKISI-Nle-WQ; X¹X²X³-(I/L/V)-KKISI-Nle-WR; X¹X²X³-(I/L/V)-KKIFI-Nle-WQ; X¹X²X³-(I/L/V)-KKIFI-Nle-WR; X¹X²X³-(I/L/V)-KIISI-Nle-WQ; X¹X²X³-(I/L/V)-KIISI-Nle-WR; X¹X²X³-(I/L/V)-KIIFI-Nle-WQ; or X¹X²X³-(I/L/V)-KIIFI-Nle-WR.

The peptide can comprise, consist essentially of, or consist of any one of the consensus sequences: X¹X²K-(I/L/V)-KQIX⁸IX¹⁰WX¹²; X¹X²K-(I/L/V)-KKIX⁸IX¹⁰WX¹²; X¹X²K-(I/L/V)-KIIX⁸IX¹⁰WX¹²; X¹X²K-(I/L/V)-KX⁶ISIX¹⁰WX¹²; X¹X²K-(I/L/V)-KX⁶IFIX¹⁰WX¹²; X¹X²K-(I/L/V)-KX⁶IX⁸I-Nle-WX¹²; X¹X²K-(I/L/V)-KX⁶IX⁸IX¹⁰WQ; X¹X²K-(I/L/V)-KX⁶IX⁸IX¹⁰WR; X¹X²KX⁴K-(Q/K/I)-ISIX¹⁰WX¹²; X¹X²KX⁴K-(Q/K/I)-IFIX¹⁰WX¹²; X¹X²KX⁴K-(Q/K/I)-IX⁸I-Nle-WX¹²; X¹X²KX⁴K-(Q/K/I)-IX⁸IX¹⁰WQ; X¹X²KX⁴K-(Q/K/I)-IX⁸IX¹⁰WR; X¹X²KX⁴KX⁶I-(S/F)-I-Nle-WX¹²; X¹X²KX⁴KX⁶I-(S/F)-IX¹⁰WQ; X¹X²KX⁴KX⁶I-(S/F)-IX¹⁰WR; X¹X²KX⁴KX⁶IX⁸I-Nle-WQ; X¹X²KX⁴KX⁶IX⁸I-Nle-WR; X¹X²K-(I/L/V)-KQISIX¹⁰WX¹²; X¹X²K-(I/L/V)-KQIFIX¹⁰WX¹²; X¹X²K-(I/L/V)-KKISIX¹⁰WX¹²; X¹X²K-(I/L/V)-KKIFIX¹⁰WX¹²; X¹X²K-(I/L/V)-KIISIX¹⁰WX¹²; X¹X²K-(I/L/V)-KIIFIX¹⁰WX¹²; X¹X²K-(I/L/V)-KX⁶ISI-Nle-WX¹²; X¹X²K-(I/L/V)-KX⁶IFI-Nle-WX¹²; X¹X²KX⁴K-(Q/K/I)-ISIX¹⁰WQ; X¹X²KX⁴K-(Q/K/I)-ISIX¹⁰WR; X¹X²KX⁴K-(Q/K/I)-IFIX¹⁰WQ; X¹X²KX⁴K-(Q/K/I)-IFIX¹⁰WR; X¹X²K-(I/L/V)-KX⁶IX⁸I-Nle-WQ; X¹X²K-(I/L/V)-KX⁶IX⁸I-Nle-WR; X¹X²KX⁴K-(Q/K/I)-ISI-Nle-WX¹²; X¹X²KX⁴K-(Q/K/I)-IFI-Nle-WX¹²; X¹X²KX⁴K-(Q/K/I)-ISIX¹⁰WQ; X¹X²KX⁴K-(Q/K/I)-ISIX¹⁰WR; X¹X²KX⁴K-(Q/K/I)-IFIX¹⁰WQ; X¹X²KX⁴K-(Q/K/I)-IFIX¹⁰WK; X¹X²KX⁴K-(Q/K/I)-IX⁸I-Nle-WQ; X¹X²KX⁴K-(Q/K/I)-IX⁸I-Nle-WR; X¹X²KX⁴KX⁶I-(S/F)-I-Nle-WQ; X¹X²KX⁴KX⁶I-(S/F)-I-Nle-WR; X¹X²K-(I/L/V)-KQISI-Nle-WX¹²; X¹X²K-(I/L/V)-KQIFI-Nle-WX¹²; X¹X²K-(I/L/V)-KKISI-Nle-WX¹²; X¹X²K-(I/L/V)-KKIFI-Nle-WX¹²; X¹X²K-(I/L/V)-KIISI-Nle-WX¹²; X¹X²K-(I/L/V)-KIIFI-Nle-WX¹²; X¹X²K-(I/L/V)-KQISIX¹⁰WQ; X¹X²K-(I/L/V)-KQISIX¹⁰WR; X¹X2K-(I/L/V)-KQIFIX¹⁰WQ; X¹X²K-(I/L/V)-KQIFIX¹⁰WR; X¹X²K-(I/L/V)-KKISIX¹⁰WQ; X¹X²K-(I/L/V)-KKISIX¹⁰WR; X¹X²K-(I/L/V)-KKIFIX¹⁰WQ; X¹X²K-(I/L/V)-KKIFIX¹⁰WR; X¹X²K-(I/L/V)-KIISIX¹⁰WQ; X¹X²K-(I/L/V)-KIISIX¹⁰WR; X¹X²K-(I/L/V)-KIIFIX¹⁰WQ; X¹X²K-(I/L/V)-KIIFIX¹⁰WR; X¹X²K-(I/L/V)-KX⁶ISI-Nle-WQ; X¹X²K-(I/L/V)-KX⁶ISI-Nle-WR; X¹X²K-(I/L/V)-KX⁶IFI-Nle-WQ; X¹X²K-(I/L/V)-KX⁶IFI-Nle-WR; X¹X²KX⁴K-(Q/K/I)-ISI-Nle-WQ; X¹X²KX⁴K-(Q/K/I)-ISI-Nle-WR; X¹X²KX⁴K-(Q/K/)-IFI-Nle-WQ; X¹X²KX⁴K-(Q/K/I)-IFI-Nle-WR; X¹X²K-(I/L/V)-KQISI-Nle-WQ; X¹X²K-(I/L/V)-KQISI-Nle-WR; X¹X²K-(I/L/V)-KQIFI-Nle-WQ; X¹X²K-(I/L/V)-KQIFI-Nle-WR; X¹X²K-(I/L/V)-KKISI-Nle-WQ; X¹X²K-(I/L/V)-KKISI-Nle-WR; X¹X²K-(I/L/V)-KKIFI*-*Nle-WQ; X¹X²K-(IL/V)-KKIFI-Nle-WR; X¹X²K-(I/L/V)-KIISI-Nle-WQ; X¹X²K-(I/L/V)-KIISI-Nle-WR; X¹X²K-(I/L/V)-KIIFI-Nle-WQ; or X¹X²K-(I/L/V)-KIIFI-Nle-WR.

In all the above embodiments, X¹ may be Pyr, H, P or R and/or X² may be S, Dpr, H, G or A. In preferred embodiments thereof, X¹ is Pyr, H, P or R and X² is S, Dpr, H, G or A.

In various embodiments, the peptide comprises any one of the following amino acid sequences:
(1)

   X¹X²KX⁴KX⁶IX⁸IX¹⁰WX¹²;
(2)

   X¹X²KIKX⁶IX⁸IX¹⁰WX¹²;
(3)

   X¹X²KLKX⁶IX⁸IX¹⁰WX¹²;
(4)

   X¹X²KVKX⁶IX⁸IX¹⁰WX¹²;
(5)

   X¹X²KX⁴KQIX⁸IX¹⁰WX¹²;
(6)

   X¹X²KX⁴KKIX⁸IX¹⁰WX¹²;
(7)

   X¹X²KX⁴KIIX⁸1X¹⁰WX¹²
(8)

   X¹X²KX⁴KX⁶ISIX¹⁰WX¹²;
(9)

   X¹X²KX⁴KX⁶IFIX¹⁰WX¹²;
(10)

   X¹X²KX⁴KX⁶IX⁸I-Nle-WX¹²;
(11)

   X¹X²KX⁴KX⁶IX⁸IX¹⁰WQ;
(12)

   X¹X²KX⁴KX⁶IX⁸IX¹⁰WR;
(13)

   X¹X²KX⁴KX⁶IX⁸IX¹⁰WH;
(14)

   X¹X²KIKQISIX¹⁰X¹¹X¹²; or
(15)

   X¹X²KIKQISIX¹⁰WX¹².
In various embodiments of these sequences (1) to (9) and (11) to (15) X¹⁰ is Nle. In various embodiments of these sequences (1) to (9) and (14) to (15) X¹⁰ is Nle and X¹² is Q. In various embodiments of these sequences (1) to (9) and (14) to (15) X¹⁰ is Nle and X¹² is R. In various embodiments of these sequences (1) to (9) and (14) to (15) X¹⁰ is Nle and X¹² is H. In various embodiments of (1) to (15) X¹ is Pyr, H, P or R. In various embodiments of (1) to (15) X² is S, Dpr, H, G or A. In various embodiments of (1) to (15), X¹ is Pyr, H, P or R and X² is S, Dpr, H, G or A. In various embodiments of (1) to (9) and (11) to (15), X¹ is Pyr, H, P or R and X² is S, Dpr, H, G or A and X¹⁰ is Nle. In various embodiments of (1) to (9) and (14) to (15), X¹ is Pyr, H, P or R and X² is S, Dpr, H, G or A and X¹⁰ is Nle and X¹² is Q. In various embodiments of (1) to (9) and (14) to (15), X¹ is Pyr, H, P or R and X² is S, Dpr, H, G or A and X¹⁰ is Nle and X¹² is R. In various embodiments of (1) to (9) and (14) to (15), X¹ is Pyr, H, P or R and X² is S, Dpr, H, G or A and X¹⁰ is Nle and X¹² is H.

The peptide of the invention may thus comprise the amino acid sequence:

X¹X²KIKQISIX¹⁰WX¹²

wherein X¹⁰ is preferably Nle and/or X¹² is preferably Q. The amino acid sequenced comprised in the peptides of the invention may thus be X¹X²KIKQISIX¹⁰WQ, X¹X²KIKQISI-Nle-WX¹² or X¹X² KIKQISI-Nle-WQ. In all these embodiments, X¹ may be Pyr, H, P or R and/or X² may be S, Dpr, H, G or A. In preferred embodiments thereof, X¹ is Pyr, H, P or R and X² is S, Dpr, H, G or A.

In various embodiments, the peptide comprises, consists essentially of or consists of the amino acid sequence:
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4), wherein the N-terminus is acetylated;
HSKIKQISI-Nle-WQ (SEQ ID NO:5);
Pyr-Hse-KIKQISI-Nle-WQ (SEQ ID NO:6);
Pyr-Dpr-KIKQISI-Nle-WQ (SEQ ID NO:7);
H-Dpr-KIKQISI-Nle-WQ (SEQ ID NO:8);
R-Dpr-KIKQISI-Nle-WQ (SEQ ID NO:9);
KSKIKQISI-Nle-WQ (SEQ ID NO:10), wherein the N-terminus is acetylated;
RSKIKQISI-Nle-WQ (SEQ ID NO:11), wherein the N-terminus is acetylated;
Pyr-SKIKKISI-Nle-WQ (SEQ ID NO:12);
Pyr-SKIKQISI-Nle-WR (SEQ ID NO:13);
Orn-SKIKQISI-Nle-WQ (SEQ ID NO:14), wherein the N-terminus is acetylated;
RSKIKQISI-Nle-WQ (SEQ ID NO:15);
HCKIKQISI-Nle-WQ (SEQ ID NO:16);
RHKIKQISI-Nle-WQ (SEQ ID NO:17);
RCKIKQISI-Nle-WQ (SEQ ID NO:18);
RSRIKQISI-Nle-WQ (SEQ ID NO:19);
Pyr-SKIKQISI-Nle-WQ (SEQ ID NO:20);
ASKIKQISI-Nle-WQ (SEQ ID NO:21), wherein the N-terminus is acetylated;
GSKIKQISI-Nle-WQ (SEQ ID NO:22), wherein the N-terminus is acetylated;
HSKIKQISI-Nle-WQ (SEQ ID NO:23), wherein the N-terminus is acetylated;
NSKIKQISI-Nle-WQ (SEQ ID NO:24), wherein the N-terminus is acetylated;
PSKIKQISI-Nle-WQ (SEQ ID NO:25), wherein the N-terminus is acetylated;
QSKIKQISI-Nle-WQ (SEQ ID NO:26), wherein the N-terminus is acetylated;
SSKIKQISI-Nle-WQ (SEQ ID NO:27), wherein the N-terminus is acetylated;
TSKIKQISI-Nle-WQ (SEQ ID NO:28), wherein the N-terminus is acetylated;
Pyr-AKIKQISI-Nle-WQ (SEQ ID NO:29);
Pyr-GKIKQISI-Nle-WQ (SEQ ID NO:30);
Pyr-HKIKQISI-Nle-WQ (SEQ ID NO:31);
Pyr-NKIKQISI-Nle-WQ (SEQ ID NO:32);
Pyr-PKIKQISI-Nle-WQ (SEQ ID NO:33);
Pyr-SRIKQISI-Nle-WQ (SEQ ID NO:34);
Pyr-SKFKQISI-Nle-WQ (SEQ ID NO:35);
Pyr-SKKKQISI-Nle-WQ (SEQ ID NO:36);
Pyr-SKVKQISI-Nle-WQ (SEQ ID NO:37);
Pyr-SKWKQISI-Nle-WQ (SEQ ID NO:38);
Pyr-SKIRQISI-Nle-WQ (SEQ ID NO:39);
Pyr-SKIKRISI-Nle-WQ (SEQ ID NO:40);
Pyr-SKIKSISI-Nle-WQ (SEQ ID NO:41);
Pyr-SKIKQIMI-Nle-WQ (SEQ ID NO:42);
Pyr-SKIKQISIFWQ (SEQ ID NO:43);
Pyr-SKIKQISIIWQ (SEQ ID NO:44);
Pyr-SKIKQISILWQ (SEQ ID NO:45);
Pyr-SKIKQISIMWQ (SEQ ID NO:46);
Pyr-SKIKQISIQWQ (SEQ ID NO:47);
Pyr-SKIKQISITWQ (SEQ ID NO:48);
Pyr-SKIKQISIWWQ (SEQ ID NO:49);
Pyr-SKIKQISIYWQ (SEQ ID NO:50);
Pyr-SKIKQISI-Nle-FQ (SEQ ID NO:51);
Pyr-SKIKQISI-Nle-WH (SEQ ID NO:52);
Pyr-SKIKQISI-Nle-WK (SEQ ID NO:53);
Pyr-SKIKQISI-Nle-WS (SEQ ID NO:54);
KGGQSKIKQISI-Nle-WQ (SEQ ID NO:55), wherein the N-terminus is acetylated;
KGQSKIKQISI-Nle-WQ (SEQ ID NO:56), wherein the N-terminus is acetylated;
KQSKIKQISI-Nle-WQ (SEQ ID NO:57), wherein the N-terminus is acetylated;
KISKIKQISI-Nle-WQ (SEQ ID NO:58), wherein the N-terminus is acetylated;
HHQSKIKQISI-Nle-WQ (SEQ ID NO:59), wherein the N-terminus is acetylated;
Pyr-SKIKQISI-Nle-WQHH (SEQ ID NO:60);
SKIKQISI-Nle-WQ (SEQ ID NO:61), wherein the N-terminus is acetylated;
Pyr-KIKQISI-Nle-WQ (SEQ ID NO:62);
ASKIKQISI-Nle-WQ (SEQ ID NO:63);
DSKIKQISI-Nle-WQ (SEQ ID NO:64);
ESKIKQISI-Nle-WQ (SEQ ID NO:65);
FSKIKQISI-Nle-WQ (SEQ ID NO:66);
GSKIKQISI-Nle-WQ (SEQ ID NO:67);
ISKIKQISI-Nle-WQ (SEQ ID NO:68);
KSKIKQISI-Nle-WQ (SEQ ID NO:69);
LSKIKQISI-Nle-WQ (SEQ ID NO:70);
MSKIKQISI-Nle-WQ (SEQ ID NO:71);
NSKIKQISI-Nle-WQ (SEQ ID NO:72);
QSKIKQISI-Nle-WQ (SEQ ID NO:73);
SSKIKQISI-Nle-WQ (SEQ ID NO:74);
TSKIKQISI-Nle-WQ (SEQ ID NO:75);
VSKIKQISI-Nle-WQ (SEQ ID NO:76);
WSKIKQISI-Nle-WQ (SEQ ID NO:77);
YSKIKQISI-Nle-WQ (SEQ ID NO:78);
Pyr-Abu-KIKQISI-Nle-WQ (SEQ ID NO:79);
Pyr-AKIKQISI-Nle-WQ (SEQ ID NO:80);
Pyr-SKIKQISI-Nva-WQ (SEQ ID NO:81);
Pyr-SKIKQISI-Gly(C5)-WQ (SEQ ID NO:82);
Pyr-SKIKQISI-Gly(C6)-WQ (SEQ ID NO:83);
Me2Q-SKIKQISI-Nle-WQ (SEQ ID NO:84);
Dpr-bAla-QSKIKQISI-Nle-WQ (SEQ ID NO:85); or
Pyr-CKIKQISI-Nle-WQ (SEQ ID NO:86).

In various embodiments, the peptide comprises, consists essentially of or consists of the amino acid sequence
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4), wherein the N-terminus is acetylated;
KSKIKQISI-Nle-WQ (SEQ ID NO:10), wherein the N-terminus is acetylated;
RSKIKQISI-Nle-WQ (SEQ ID NO:11), wherein the N-terminus is acetylated;
Pyr-SKIKKISI-Nle-WQ (SEQ ID NO:12);
Pyr-SKIKQISI-Nle-WR (SEQ ID NO:13);
Orn-SKIKQISI-Nle-WQ (SEQ ID NO:14), wherein the N-terminus is acetylated;
HSKIKQISI-Nle-WQ (SEQ ID NO:5);
RSKIKQISI-Nle-WQ (SEQ ID NO:15);
Pyr-Hse-KIKQISI-Nle-WQ (SEQ ID NO:6);
Pyr-Dpr-KIKQISI-Nle-WQ (SEQ ID NO:7);
H-Dpr-KIKQISI-Nle-WQ (SEQ ID NO:8);
HCKIKQISI-Nle-WQ (SEQ ID NO:16);
RHKIKQISI-Nle-WQ (SEQ ID NO:17);
R-Dpr-KIKQISI-Nle-WQ (SEQ ID NO:9);
RCKIKQISI-Nle-WQ (SEQ ID NO:18);
RSRIKQISI-Nle-WQ (SEQ ID NO:19);
HSKIKQISI-Nle-WQ (SEQ NO:23), wherein the N-terminus is acetylated;
PSKIKQISI-Nle-WQ (SEQ NO:25), wherein the N-terminus is acetylated;
Pyr-AKIKQISI-Nle-WQ (SEQ NO:29);
Pyr-GKIKQISI-Nle-WQ (SEQ NO:30);
Pyr-HKIKQISI-Nle-WQ (SEQ NO:31);
Pyr-SKVKQISI-Nle-WQ (SEQ NO:37);
KQSKIKQISI-Nle-WQ (SEQ NO:57), wherein the N-terminus is acetylated;
Pyr-KIKQISI-Nle-WQ (SEQ NO:62);
ASKIKQISI-Nle-WQ (SEQ NO:63);
LSKIKQISI-Nle-WQ (SEQ NO:70);
MSKIKQISI-Nle-WQ (SEQ NO:71);
QSKIKQISI-Nle-WQ (SEQ NO:73);
SSKIKQISI-Nle-WQ (SEQ NO:74);
YSKIKQISI-Nle-WQ (SEQ NO:78);
Pyr-Abu-KIKQISI-Nle-WQ (SEQ NO:79);
Pyr-AKIKQISI-Nle-WQ (SEQ NO:80); or
Pyr-CKIKQISI-Nle-WQ (SEQ NO:86).

In various embodiments, the peptide comprises, consists essentially of or consists of the amino acid sequence
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4) wherein the N-terminus is acetylated;
HSKIKQISI-Nle-WQ (SEQ ID NO:5);
Pyr-Hse-KIKQISI-Nle-WQ (SEQ ID NO:6);
Pyr-Dpr-KIKQISI-Nle-WQ (SEQ ID NO:7);
H-Dpr-KIKQISI-Nle-WQ (SEQ ID NO:8); or
R-Dpr-KIKQISI-Nle-WQ (SEQ ID NO:9).

In some embodiments, the peptide comprises, consists essentially of or consists of the amino acid sequence
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3); or
RSRIKQISI-Nle-WQ (SEQ ID NO:4), wherein the N-terminus is acetylated.

The peptide of the invention may comprise the given sequence, essentially consist of it or may even consist of it. "Essentially consist of", as used in this context, means that the peptide comprises only 1-10 additional amino acids (in addition to the given sequence), which may form the N-terminal and/or C-terminal flanking sequence, as defined below.

If the peptide comprises the given sequence this means that the given sequence may be part of a longer peptide that may be up to 100 amino acids in length. In various embodiments, the peptide is up to 90, up to 80, up to 70, up to 60 or up to 50 amino acids in length. In various embodiments, the upper limit may be 50, 49, 48, 47, 46, 45, 44, 43, 42, 41, 40, 39, 38, 37, 36, 35, 34, 33, 32, 31 or 30 amino acids. The peptide may thus be 10 to 30 amino acids in length, for example 10 to 29, 10 to 28, 10 to 27, 10 to 26, 10 to 25, 10 to 24, 10 to 23, 10 to 22, 10 to 21 or preferably 10 to 20 amino acids in length. The peptide may be even shorter, for example 10 to 19, 10 to 18, 10 to 17, 10 to 16, 10 to 15, 10 to 14, 10 to 13 or 10 to 12 amino acids in length. The peptide may also have a lower limit length of 10, 11 or 12 amino acids. In various embodiments, the peptide is 10, 11, 12, 13, 14 or 15 amino acids in length.

All peptides of the invention comprise the amino acid sequence of X¹ to X¹² as defined herein, with X¹ and/or X² optionally being absent, but may comprise additional amino acid sequences that flank the given core sequence on its N- and/or C-terminus. The peptide may thus comprise in addition to the amino acid sequence of X¹ to X¹² as defined herein an N-terminal flanking sequence and/or a C-terminal flanking sequence. The flanking sequences may independently be of any length, as long as the resulting peptide meets the length limitations defined herein.

In various embodiments, the peptide may comprise an N-terminal flanking sequence that is 0 to 15 amino acids in length, such as 1, 2, 3, 4, 5, 6, 7, 8, 9, 10, 11, 12, 13, 14, or 15 amino acids, for example 1 to 10, 1 to 9, 1 to 8, 1 to 7, 1 to 6, 1 to 5, 1 to 4, 1 to 3, or 1 to 2 amino acids in length. The N-terminal flanking sequence is linked to X¹ or, if X¹ is absent, to X² or, if X² is also absent, to X³, by a peptide bond that connects the free amino terminus of X¹, X² or X³ with the C-terminus of the N-terminal flanking sequence. The N-terminal flanking sequence may be K, KG, KGG, HH, H, Me2Q, Dpr, Dpr-bAla, and bAla.

In various embodiments, the peptide may comprise a C-terminal flanking sequence that is 0 to 15 amino acids in length, such as 1, 2, 3, 4, 5, 6, 7, 8, 9, 10, 11, 12, 13, 14, or 15 amino acids, for example 1 to 10, 1 to 9, 1 to 8, 1 to 7, 1 to 6, 1 to 5, 1 to 4, 1 to 3, or 1 to 2 amino acids in length. The C-terminal flanking sequence is linked to X¹² by a peptide bond that connects the free carboxy terminus of X¹² with the N-terminus of the C-terminal flanking sequence.

In various embodiments, the peptide may comprise an N-terminal flanking sequence that is 0 to 15 amino acids in length, such as 1, 2, 3, 4, 5, 6, 7, 8, 9, 10, 11, 12, 13, 14, or 15 amino acids, for example 1 to 10, 1 to 9, 1 to 8, 1 to 7, 1 to 6, 1 to 5, 1 to 4, 1 to 3, or 1 to 2 amino acids in length and a C-terminal flanking sequence that is 0 to 15 amino acids in length, such as 1, 2, 3, 4, 5, 6, 7, 8, 9, 10, 11, 12, 13, 14, or 15 amino acids, for example 1 to 10, 1 to 9, 1 to 8, 1 to 7, 1 to 6, 1 to 5, 1 to 4, 1 to 3, or 1 to 2 amino acids in length. In various embodiments, the N-terminal flanking sequence is 1 to 10, for example 1 to 5 amino acids in length and the C-terminal flanking sequence is also 1 to 10 or 1 to 5 amino acids in length. In various of these embodiments, the N-terminal flanking sequence may be any one of K, KG, KGG, HH, H, Me2Q, Dpr, Dpr-bAla, and bAla.

It is generally preferred for various reasons, such as ease of synthesis and handling, that the peptide is as short as possible without impairing its functionality. It may thus be preferred, in various embodiments, that the flanking sequences are as short as possible or even absent. In such embodiments, the peptides consist of the given sequence.

It has been described above that the peptide may have a protected N-terminus, with exemplary protecting groups listed herein above. Similarly, the C-terminus of the peptide may also be modified as desired. Possible modifications include, without limitation, an amidation of the C-terminal carboxy group.

According to various embodiments, although not preferred, the invention also encompasses retro, inverso or retroinverso derivatives of the peptides defined above, which retain the transduction promoting properties herein disclosed. "Retaining the transduction promoting properties", as used in this context, means that the respective retains at least 80% of the transduction promoting functionality of the corresponding template peptide.

As also described herein above, the peptides of the invention may comprise one or more D amino acids. It is however preferred that all amino acids are in the L form.

Further embodiments of the invention are peptides derived from those disclosed herein above which comprise at least one of the following covalent modifications:
- linkage to a non-peptidic macro molecular carrier group; preferably at the N-teminus;
- linkage to a non-peptidic macro molecular carrier group; preferably at the C- terminus;
- glycosylation; preferably at amino acid side chains;
- linkage to an adaptor molecule, which promotes uptake of the peptide into cells, or linkage to a hydrophobic group, preferably a lipid, a fatty acid, a dansyl, a carboxyfluorescein, a carbobenzoxyl (benzyloxycarbonyl; Cbz) or a t- butyloxycarbonyl group;
- oxidation, sulphatization, esterification, lactone formation and/or phosphorylation.

The invention also covers multimers, for example dimers or trimers, of the peptides described above. In the context of the present invention, a "multimer" denotes functional peptides as disclosed herein above that have been covalently linked together. Dimers, which are two functional peptides linked together, may be for example obtained by introduction of thiol groups at the C or N-terminus - these groups can then be used to generate dimers by formation of a disulfide bridge. Other reagents can of course also be used to generate such multimers.

In various embodiments, the peptides of the invention may also comprise the amino acid sequences disclosed herein multiple times, such as twice or thrice, for example directly adjacent to each other or linked by a linker peptide sequence. The length limitations disclosed herein still apply to such peptides.

Suitable macromolecular carrier groups for the peptides of the invention include, but are not limited to, polyethylene glycol (PEG), polyoxyalkylene glycol, polysorbate esters, mannan, amylopectin, pullulan, hydrogel nanoparticles of self-aggregated hydrophobized polysaccharides, polylysine, antibodies, antibody-like molecules, antibody fragments, or albumine.

The invention also relates to peptide mimetics of the peptides according to the invention. These can be characterized for example by a modification of one or more peptide bonds, for example, by a reverse peptide bond or by an ester bond.

All peptides disclosed herein may be in form of suitable salts, depending on their sequence. Such salts of the peptides of the invention are also encompassed by the present invention. Typical salt forms of the peptides of the invention include but are not limited to the counter ions trifluoroacetic acid (TFA), chloride, acetate, sulfate, phosphate, maleate, fumarate, lactate, tartrate, citrate and gluconate, hydrochloride, hydrobromide, sodium, potassium, and ammonium.

The present invention also covers methods for the productions of the peptides disclosed herein. These methods include all known and conventionally used methods for peptide production and include chemical synthesis as well as recombinant production. The invention also includes (methods for) the *in situ* generation of the claimed peptides from suitable precursors.

According to another aspect, the invention also provides nucleic acids coding for the peptides of the invention and expression vectors for the inventive peptides, such as plasmids, cosmids and viral vectors. The nucleic acid encoding the peptides according to the invention may be DNA, RNA, genomic DNA or PNA. The invention also relates to vectors containing the polynucleotide according to the invention, and genetically engineered host cells containing the vector according to the invention.

The peptides of the invention promote viral infection of a cell. More specifically, the peptides of the invention have the ability to improve the transduction efficiency of a virus or viral vector, typically relative to the same virus or viral vector in the absence of the peptide. The transduction efficiency can be determined by routine means and assays known to those skilled in the art and exemplified herein below in the examples. The viruses or viral vectors may be selected from those disclosed herein below, but in various embodiments are retroviruses, in particular gamma-retroviruses or lentiviruses.

As used herein, "viruses" generally relates to natural occurring viruses as well as artificial viruses; respective viral vectors. Viruses in the sense of the present invention include, but are not limited to, paramyxoviruses (such as respiratory syncytial virus, measle virus), orthomyxoviruses (such as influenza virus), flaviviruses (such as hepatitis C virus), hepadnaviruses (such as hepatitis B virus), rhabdoviruses (such as rabies, VSV), coronaviruses (such as SARS), togaviruses (such as sindbis virus, chikungunya virus), filoviruses (such as ebola virus), parvoviruses (such as adeno-associated viruses), adenoviruses, arenaviruses, poxviruses, herpesviruses, bunyaviruses, bornaviruses, arteriviruses, and baculoviruses.

According to a particular embodiment, the viruses are artificial viruses; or viral vectors, which may for instance comprise a nucleic acid designed for gene transfer, in particular gene and/or cell therapy. In various embodiments, the viruses are enveloped viruses. In various embodiments, the viruses are retroviruses and in particular gamma-retroviruses or lentiviruses. The viral vectors could have pseudotyped envelopes with different glycoproteins from vesicular stomatis virus (VSV), modified feline endogenous retrovirus (RD114), amphotropic murine leukemia virus (MLV), modified gibbon ape leukemia virus (GALV), modified baboon envelope retrovirus (BaEV) and even with glycoproteins from AcMNPV baculovirus (GP64), the latter being a virus normally specific for insect cells.

The invention also relates to compositions comprising one or more peptides of the invention. Composition comprising at least one peptide as described herein thus also form part of the invention. The composition may be a pharmaceutical composition. In other embodiments, the composition is a non-therapeutic composition. In all embodiments of such compositions described herein, the peptides of the invention may be present in form of their salts, in particular pharmaceutically acceptable salts.

If the composition is a pharmaceutical composition, it may comprise a peptide as defined herein and a suitable pharmaceutically acceptable auxiliary, such as a carrier or solvent. Pharmaceutical compositions according to the invention can also contain pharmaceutically acceptable auxiliary agents which contribute, for example, to the solubility, stability or sterility of the composition or increase the efficiency of uptake into the body.

The form and content of the pharmaceutical composition which contains the peptide(s) depends on the route of administration. Preferably, galenic formulations and application forms are selected in which the peptide(s) arrive(s) at the target site in a non-degraded condition. The medicament/biopharmaceutical can be administered locally as injection, drops, spray, tablets, suppositories, cream, ointments, gel etc.

The compositions of the invention comprising at least one peptide of the invention may also be culture mediums, said composition being intended for use as an infection promoting reagent for facilitating the transduction of a cell with a virus or viral vector, in particular an enveloped virus or viral vector. Thus, the invention also relates to a virus infection promoting reagent comprising at least one peptide according to the present invention, in a suitable medium, in particular in a suitable culture medium.

The compositions of the invention may further comprise at least one virus or viral vector, as described herein.

According to a further aspect, the invention relates to a complex of at least one peptide of the invention with at least one virus particle, for example an enveloped virus particle, such as an enveloped viral vector for gene therapy.

The invention also relates to a mixture of at least one peptide of the invention with at least one virus particle, for example an enveloped virus particle, such as an enveloped viral vector for gene therapy, and at least one cell.

The peptide, mixture, complex or pharmaceutical composition or medicament/biopharmaceutical of the invention can be administered *in vivo,* for example by injecting it via the intramuscular, intravenous, intraarterial, intra-peritoneal or intracranial route. It is possible to perform the administration as a bolus or repeatedly over a period of time.

The invention also covers methods for preparing the above-described compositions, complexes and mixtures. In various embodiments, said methods comprise mixing the at least one peptide with at least one viral particle and/or at least one cell.

In still another aspect, the invention provides a kit comprising a peptide as defined above and a virus or viral vector.

The peptides of the invention can be used as a general means for increasing transduction efficiencies of viruses in eukaryotic cells, in particular enveloped viruses.

The invention thus covers the use of at least one peptide of the invention or composition, mixture or complex comprising such peptide for promoting the infection of a eukaryotic cell by a virus or viral vector.

Said "promoting" relates to a measurable transduction efficacy, in particular in relation to the transduction in the absence of the peptide or any other known transduction enhancer. Said efficacy may be expressed as an increase of at least 10% relative to the transduction without the peptide, preferably at least 20%, more preferably at least 30% or at least 40% or at least 50%.

In various embodiments, the peptides and compositions of the invention are for use in transducing a eukaryotic cell. Such use may be therapeutic or non-therapeutic. In the latter case, the invention thus also features (non-therapeutic) use of the peptides and compositions of the invention are for transducing a eukaryotic cell, for example a cultured cell.

In various embodiments, the peptides and compositions of the invention are for use in gene and/or cell therapy, in particular the generation of therapeutic cell products by gene transfer. The peptides described herein can, for example and without limitation, be used in the production of CAR T-cells.

An aspect of the invention therefore relates to a peptide of the invention or a composition comprising one or more peptides of the invention or the above described complexes and mixtures for use as a medicament or biopharmaceutical. The medicament or biopharmaceutical may be used for increasing the efficiency of a gene therapy viral vector.

The (target) cells can be any kind of eukaryotic cells such as mammalian cells, in particular human, mouse, rat, monkey, dog or hamster cells. Representative, non-limiting, target tissues/cells are hematopoietic cells, skin, muscle, liver, eye, neurons, lymphocytes, fibroblasts, keratinocytes, adipocytes, myoblasts, hepatocytes, tumor cells and more generally any eukaryotic cell that is known or will be identified as a target of a virus. In various embodiments, the target cells are leukocytes, in particular lymphocytes such as T cells or NK cells.

The peptides of the invention may be used as cell penetrating peptides. In particular, the peptide may be used as a delivery system for bioactive compounds such as nucleic acid, for example plasmid DNA, siRNAs, shRNA, antisense oligonucleotides, and other bioactive compounds (peptides or proteins, in particular therapeutic peptides or proteins, marker peptides, antibodies, etc.). The cell penetrating peptides can be either covalently or non-covalently linked to the bioactive compound.

The peptides of the invention may also be used as immune adjuvants. In particular, the peptides may be covalently bound to an immune epitope and thus form a vaccine.

The invention also relates to methods for the infection of a eukaryotic cell by a virus or viral vector, wherein said method comprises the step of contacting the cell with the virus or viral vector in the presence of at least one peptide, composition, complex or mixture according to the invention. The method may also comprise the step of providing the peptide, composition, mixture or complex.

Further aspects relate to methods for increasing the efficiency of a nucleic acid transfer into a eukaryotic target cell with a viral vector, comprising contacting the target cell with the viral vector in the presence of at least one peptide according to the invention or composition, complex or mixture comprising it. The method may also comprise the step of providing the peptide, composition, mixture or complex. "Increasing the transduction efficiency", as used in this context, relates to a measurable increase in transduction efficacy, for example an increase of at least 10% relative to the transduction without the peptide or any other transduction enhancer, preferably at least 20%, more preferably at least 30% or at least 40% or at least 50%. The transduction activity and efficacy of the peptides with a given virus and given target cell can be measured using a reporter assay, for instance using a luciferase assay or a GFP expression assay as exemplarily demonstrated in the examples.

The invention also relates to a method for gene therapy, comprising administering to a patient in need thereof a peptide, complex, mixture or pharmaceutical composition as described above. The method comprises also administering a virus vector for gene therapy before, after or together with the administration of the peptide of the invention.

In the uses and methods of the present invention, the peptides as described herein are typically used in an effective amount. The term "effective amount", as used herein in relation with the peptides, means the amount required for increasing the transduction efficiency of a viral vector.

The concrete effective amount will generally depend on the particular peptide tested, the target cell and the viral vector implemented. This amount can be determined according to methods well known in the art, in particular according to the methods illustrated in the examples.

In various embodiments, the peptides of the invention may be combined with another transduction improving means. For example, in a particular embodiment, transduction efficiency is increased by using at least one peptide according to the invention and at least one further transduction enhancer different from these peptides of the invention, such as fibronectin and derivates thereof, PTD-B; a SEVI peptide and amphiphilic/cationic polymers like polaxamers or hexadimethrine bromide. This applies to the compositions, mixtures, complexes, methods and uses of the invention.

In another aspect, the invention also features a (eukaryotic) cell transduced with at least one peptide of the present invention or a composition, mixture or complex comprising said at least one peptide of the invention. Also covered are cells obtainable or obtained by the methods described herein.

The peptides described herein are used for a broad range of therapeutic and diagnostic applications and are valuable laboratory tools for the performance and study of entry of viruses into cells.

In various embodiments of the invention a peptide of the invention is used as a general enhancer of viral infection or transduction efficiencies for routine laboratory practice or gene therapeutic approaches based on viral vector systems. The peptides are enhancing the entry of vectors designed for gene therapy into cells *in vitro, ex vivo or in vivo.* They may be administered in combination with a viral vector for gene therapy and mediate entry of the viral vector into the target cell. The peptides are also useful *in vitro* because they promote the uptake of viruses into cells. They are thus useful as a tool for studying viruses and their mechanisms of action. Another embodiment of the invention is the use of the peptides of the invention for diagnostic approaches, for example for diagnosing a disease or disorder, especially those related to viruses such as enveloped viruses. The peptides of the invention enhance the infectious titers of virus particles and therefore enhance the cellular uptake, allowing the detection of residual viral contaminations. Therefore, they can be used to isolate viral particles from samples like serum, blood, plasma, sperm or tissues derived from subjects, in particular a human subject suspected to be infected by a virus, more specifically by an enveloped virus. The peptides according to the invention can also be used to study viral particles from water, food or any other source.

The peptides of the invention can also be used to enhance gene delivery rates in *ex vivo* or *in vivo* gene therapy approaches based on vector systems, in particular on enveloped vector systems. Accordingly, the invention also relates to a peptide as described above for use in gene therapy for promoting the infection of an eukaryotic cell by a virus or a viral vector in a subject in need thereof. The peptide of the invention can be used in combination with a virus or viral vector in gene therapy. The peptide may be for the simultaneous, separate or sequential administration with the gene therapy vector. The generation of highly infectious retroviral vectors for gene therapy, especially for ex *vivo* gene therapy of stem cells, is a difficult procedure. In particular, the transduction efficiencies of retroviral vectors for stem cells are low. In the presence of the peptides of the invention, however, stem cells and cell lines can be efficiently transduced with retroviral vectors, resulting in higher efficiencies for gene delivery into the target cell compared to samples containing no peptide.

In all *in vivo* or *in vitro* methods and uses described herein, the peptides may be used in immobilized form, for example immobilized to a solid support. Advantageously, no solid support or immobilization is needed for the increase in transduction efficiency.

All embodiments disclosed herein in relation to the peptides as such, the compositions and mixtures are similarly applicable to the methods and uses of the invention and vice versa.

The invention is further described by means of the following examples.

### Examples

### Materials and methods

### Materials:

All solvents were used without further purification. Water was demineralized with a demineralisation system (Milli-Q Plus, Millipore). The reagents were purchased from Sigma-Aldrich (Deisenhofen, Germany), Bachem (Basel, Schweiz), PolyPeptide Laboratories (Strassburg, France), Merck Chemicals (Nottingham, UK), GL Biochem (Shanghai, China), VWR (Darmstadt) or IRIS Biotechnology (Marktredwitz, Germany) and used without further purification.

The vector was obtained from BIMFS (BioNTech Innovative Manufacturing Services GmbH, Idar-Oberstein), Other materials were from Miltenyi Biotech (Bergisch Gladbach) or Serva Elektrophoresis (Heidelberg).

### RP-HPLC-MS-Analysis:

Chromatography was done with an Agilent series 1200-system (2x1200 series binary pump SL G1312B, 1200 thermostatted column compartment SL G1316B, 1200 series high perf. autosampler SL G1367C, 1200 series micro-vacuum degasser G1379B, 1100/1200 automation interface G2254A, 1100/1200 well plate handler G2255A, VWD-UV detector G1314B, valve G1158A) and coupled ESI-MS (Agilent LC/MSD Quad SL system G1956B). The separation was done on a Phenomenex Gemini-NX-3u column (C18 110A, 20x2mm) at 30 °C and a flow of 1.0 ml/min with a linear gradient (5-95 % B in 6 min, with A: 0.05 % TFA in water and B: 0.05 % TFA in MeCN). UV detection was done at a wavelength of 220 nm.

### Preparative purification by HPLC:

The peptide was dissolved in MeCN/H₂O or DMSO or TFA and fractionated by HPLC. Different gradients were employed on a preparative Dionex HPLC system (Dionex Ultimate 3000 pump, Dionex Ultimate 3000 RS variable wavelength detector). UV detection was done at a wavelength of 220 nm. The following column materials were employed: Agilent PLRP-S 100A 8uM, Kromasil 100-5-C18. Waters SunFire Prep C18 OBD. This yielded the purified target peptide as TFA salt. The obtained purity was >70%, or >90% (for peptides SEQ ID NO:1 to SEQ ID NO:29).

### Abbreviations:

| | |
|---|---|
| AA | Amino acid |
| DCM | Dichloromethane |
| DIC | Diisopropylcarbodiimide |
| DMF | N,N-Dimethylformamide |
| DMSO | Dimethylsulfoxide |
| EDT | 1,2-Ethanedithiol |
| Fmoc | 9-Fluorenylmethyloxycarbonyl |
| Fmoc-AA-OH | Fmoc-protected amino acid |
| h | Hour(s) |
| min | Minutes |
| MOI | Multiplicity of infections |
| OxymaPure | Ethyl(hydroxyimino)cyanoacetate |
| HPLC | High-pressure liquid chromatography |
| SPPS | Solid-phase peptide synthesis |
| TFA | Trifluoroacetic acid |

### Peptide synthesis

The peptides were synthesized by solid phase peptide synthesis (SPPS) on polystyrene based resins. Cleavage from the resin and removal of side-chain protecting groups furnished crude peptides which were purified by RP-HPLC.

### Resin loading:

The respective C-terminal amino acid was coupled to the synthesis resin. For that, PHB resin was treated with the Fmoc-protected amino acid (0.5-5 eq.) dissolved in DCM (approx. 10 mL DCM per gram resin). Diisopropylcarbodiimide (DIC) (0.5-5 eq.) and N-Methylimidazole (NMI) (0.25-2.5 eq.) were added and gently shaken for at least 4 hours. The resin was filtrated and washed 3 times each with DMF, DCM and diethyl ether. After drying the resin in the air the loading was determined by spectrophotometry (extinction coefficient ε = 8100 cm²/mmol of the piperidin/dibenzofulven adduct obtained after resin treatment with 20% piperidine/DMF) [Eichler, J., Bienert, M., Stieranová, A., Lebl, M. Evaluation of cotton as a carrier for solid-phase peptide synthesis, Pept. Res., 1991, 4, 296-307].

### Peptide synthesis:

The amino acid loaded resin (approx. 20-80 µmol, approx. 0.2-0.3 mmol/g) was treated with a solution of piperidine in DMF (20:80, 2x 10 min) for deprotection of the Fmoc group. Following that, repeating cycles of coupling of amino acids and Fmoc cleavage were performed until the linear target peptides were assembled. For the coupling of amino acids the following reagents were employed: DIC (4.0 eq., 0.5 M in DMF), Fmoc-AA-OH (4.0 eq., 0.5 M in DMF) and OxymaPure (4.0 eq.) at RT for 90 min. For all amino acids double couplings were performed.

The following protected amino acid derivatives were employed: Fmoc-Ala-OH, Fmoc-Cys(Trt)-OH, Fmoc-Asp(OtBu)-OH, Fmoc-Glu(OtBu)-OH, Fmoc-Phe-OH, Fmoc-Gly-OH, Fmoc-His(Trt)-OH, Fmoc-Ile-OH, Fmoc-Lys(Boc)-OH, Fmoc-Leu-OH, Fmoc-Met-OH, Fmoc-Asn(Trt)-OH, Fmoc-Pro-OH, Fmoc-Gln(Trt)-OH, Fmoc-Arg(Pbf)-OH, Fmoc-Ser(tBu)-OH, Fmoc-Thr(tBu)-OH, Fmoc-Val-OH, Fmoc-Trp(Boc)-OH, Fmoc-Tyr(tBu)-OH.

### Cleavage of the peptides from the resin:

The resin-bound peptides were treated with TFA/H2O/EDT 95:3:3 or TFA/H2O/TIPS 92.5:5:2.5 or similar cleavage cocktails for 180 min. After addition of ice cold diethyl ether the suspensions were centrifuged at 4 °C and the supernatant decanted. The peptides were washed further two times with diethyl ether to furnish the crude peptides.

### Salt exchange of peptides from TFA to chloride salt:

Selected peptides, that were obtained as TFA salt after HPLC, were transferred to their corresponding chloride salt. For that, the peptide was dissolved in MeCN / H2O 50:50, 35 mM aqueous HCl (3 eq.) was added, and the peptide subjected to lyophilization. The lyophilization step was repeated to remove potential residual HCl.

### Determination of peptide solubility

Several peptides of the invention were analyzed regarding their solubility in aqueous PBS buffer. The following protocol was used:
Solution A: A stock solution of the peptide (20 µL, 10 mg/mL in DMSO) was diluted with PBS buffer (180 µL) to yield a theoretical concentration of 1 mg/mL peptide in PBS/DMSO 9:1. The resulting solution/suspension was agitated in a horizontal shaker (700 rpm) for 30 min and then centrifuged for 5 min at 13.000 rpm. An aliquot of the supernatant (50 µL) was transferred to a separate vial and diluted with DMSO (50 µL) to yield a theoretical concentration of 0.5 mg/mL peptide. LC-MS analysis with additional UV readout at a wavelength of 220 nm was performed in duplicate (4 µL, theoretically 2 µg peptide injected).
Solution B: A stock solution of the peptide (10 µL, 10 mg/mL in DMSO) was diluted with DMSO (190 µL) to yield a theoretical concentration of 0.5 mg/mL peptide. LC-MS analysis with additional UV readout at a wavelength of 220 nm was performed in duplicate (4 µL, theoretically 2 µg peptide injected).

Data analysis: The peak areas of the target peptide (detected by UV readout at 220 nm) for all measurements were determined. Division of the peptides' peak area for solution A divided by the peptides' peak area for solution B multiplied by the theoretical maximal concentration of 1000 µg/mL gave the solubility of the peptide in PBS/DMSO 9:1.

The results of the LC-MS-based solubility assay are shown in Table 1 below. The results show that the tested peptides exhibited significantly higher solubility in PBS buffer compared to known peptides.

### Determination of peptide cytotoxicity

Two exemplary peptides of the invention (SEQ ID NO:2 and SEQ ID NO:7) and a reference peptide (PTD-B; SEQ ID NO:88) were analyzed regarding their cytotoxicity in a resazurin-based cytotoxicity assay. Said assay is based on the reduction of oxidized non-fluorescent blue resazurin to the red fluorescent dye resorufin (Ex/Em of 530-560 / 590 nm) in live cells. The amount of the dye produced is directly proportional to the number of living cells.

The following protocol was used:
Logarithmic growing Jurkat cells (clone E6-1, ATCC-TIB-152) are counted and adjusted in phenol red-free medium to 2 x 10⁶ cells/ml. Subsequently, 100 µl of the cell suspension is seeded in each well of a 96-well plate (black plate with clear bottom). Peptides (10 mg/mL stock solutions in DMSO) are diluted in 100 µl medium to a final concentration of 100 µg/mL and added to the cell suspension. In addition to the peptide approaches, all controls (blank as background control, untreated cells, solvent controls,
Camptothecin treated cells and deadened cells as positive controls) are prepared in parallel. The plate is then placed in an incubator at 37°C, 5% CO₂ in 95% rH for 24 hours. Afterwards, 20 µl Resazurin solution is added to each well and incubated for 2-3 hours at 37°C. Then, the fluorescence is recorded using a microplate reader with the settings 560 nm for excitation and 590 nm for emission.

The results of the resazurin-based cytotoxicity assay were determined as viability, i.e. metabolic activity relative to cells only (which was set to 100) at a final concentration of 100 µg/mL. The results showed that the tested peptides of the invention exhibited lower cytotoxicity (viability ≥70%) compared to the reference peptide (37% viability).

### Viral vector production

Stable producer cell line (293Vec-GALV-GFP HG820-E912, described in Hennig et al., Hum Gene Ther Clin Dev, 2014 Dec;25(4):218-28. doi: 10.1089/humc.2014.083) was cultured and 4 consecutive harvests were filtered, pooled and subsequently frozen at -70°C. Resulting viral supernatants with vectors pseudotyped with GALV envelope protein, were analyzed for contaminations and titrated on indicator cells to determine the concentration of infectious particles by analyzing GFP positive cells using flow cytometry. Retroviral vectors were applied at a MOI of 0.1.

### Transduction Assay

86 exemplary peptides of the invention (SEQ ID Nos. 1-86) were analyzed regarding their transduction enhancing properties.

For this, Jurkat E6.1 cells were counted, washed and adjusted to 1.4 x 10⁶ cells/mL in RPMI (Roswell Park Memorial Institute) medium. The cells were seeded in a volume of 360 µl in a 24-well dish.

The to be tested peptides as well as a reference peptide PTD-B (SEQ ID NO:88; WO 2014/177635 A1) were dissolved in DMSO (10 mg/ml stock solution) by vigorous vortexing. Subsequently, the peptides were diluted with PBS to a working concentration of 1mg/mL and incubated for 15 min. Afterwards, the peptides were further diluted with PBS to the desired concentration (resulting in final concentrations of 5, 2.5, 1.25 and 0.625 µg/mL) and incubated with the same volume of viral vector for 15 min. 40 µl of said mixture was then added to the cells. 16-20 h after incubation of the cells at 37°C, 5% CO₂ in 95% rH, 1.6 mL culture medium including FCS was added to each well.

Read-out was done by flow cytometry after 48 h to determine dead and live cells as well as GFP fluorescence as a means for efficacy of transduction. Transduction efficacy was determined relative to the viral vector without any peptide as a reference (set to 1). A transduction efficacy of 9.21 thus means that the peptide increased the transduction 9.21-fold relative to viral vector only. The results are shown in Table 1.

**Table 1: Transduction efficacy & solubility of exemplary peptides**

| **Amino acid sequence** | **SEQ ID NO:** | **Transduction Efficacy vs. "Virus only"** | **Solubility LCMS [µg/mL]** |
|---|---|---|---|
| Pyr-SKLKQISI-Nle-WQ | SEQ ID NO:1 | 9.21 | 1143 |
| PSKIKQISI-Nle-WQ | SEQ ID NO:2 | 10.09 | 982 |
| HHKIKQISI-Nle-WQ | SEQ ID NO:3 | 4.04 | 1029 |
| Ac-RSRIKQISI-Nle-WQ | SEQ ID NO:4 | 5.20 | 1160 |
| HSKIKQISI-Nle-WQ | SEQ ID NO:5 | 9.93 | 1006 |
| Pyr-Hse-KIKQISI-Nle-WQ | SEQ ID NO:6 | 6.52 | 994 |
| Pyr-Dpr-KIKQISI-Nle-WQ | SEQ ID NO:7 | 9.09 | 1084 |
| H-Dpr-KIKQISI-NIe-WQ | SEQ ID NO:8 | 3.58 | 1028 |
| R-Dpr-KIKQISI-NIe-WQ | SEQ ID NO:9 | 3.88 | 578 |
| Ac-KSKIKQISI-Nle-WQ | SEQ ID NO:10 | 9.66 | 1123 |
| Ac-RSKIKQISI-Nle-WQ | SEQ ID NO:11 | 8.81 | 1151 |
| Pyr-SKIKKISI-Nle-WQ | SEQ ID NO:12 | 8.20 | 1350 |
| Pyr-SKIKQISI-Nle-WR | SEQ ID NO:13 | 8.47 | 810 |
| Ac-Orn-SKIKQISI-Nle-WQ | SEQ ID NO:14 | 11.21 | 1089 |
| RSKIKQISI-Nle-WQ | SEQ ID NO:15 | 9.31 | 1077 |
| HCKIKQISI-Nle-WQ | SEQ ID NO:16 | 5.72 | 129 |
| RHKIKQISI-Nle-WQ | SEQ ID NO:17 | 2.85 | 1307 |
| RCKIKQISI-Nle-WQ | SEQ ID NO:18 | 4.35 | 297 |
| RSRIKQISI-Nle-WQ | SEQ ID NO:19 | 3.79 | 87 |
| Pyr-SKIKQISI-Nle-WQ | SEQ ID NO:20 | 9.03 | 53 |
| Ac-ASKIKQISI-Nle-WQ | SEQ ID NO:21 | 4.06 | 114 |
| Ac-GSKIKQISI-Nle-WQ | SEQ ID NO:22 | 5.98 | 103 |
| Ac-HSKIKQISI-Nle-WQ | SEQ ID NO:23 | 10.92 | 48 |
| Ac-NSKIKQISI-Nle-WQ | SEQ ID NO:24 | 6.66 | 84 |
| Ac-PSKIKQISI-Nle-WQ | SEQ ID NO:25 | 9.54 | 134 |
| Ac-QSKIKQISI-Nle-WQ | SEQ ID NO:26 | 5.54 | 123 |
| Ac-SSKIKQISI-Nle-WQ | SEQ ID NO:27 | 6.10 | 137 |
| Ac-TSKIKQISI-Nle-WQ | SEQ ID NO:28 | 7.13 | 189 |
| Pyr-AKIKQISI-Nle-WQ | SEQ ID NO:29 | 10.31 | 121 |
| Pyr-GKIKQISI-Nle-WQ | SEQ ID NO:30 | 10.52 | 60 |
| Pyr-HKIKQISI-Nle-WQ | SEQ ID NO:31 | 11.69 | 53 |
| Pyr-NKIKQISI-Nle-WQ | SEQ ID NO:32 | 8.63 | 51 |
| Pyr-PKIKQISI-Nle-WQ | SEQ ID NO:33 | 9.25 | 141 |
| Pyr-SRIKQISI-Nle-WQ | SEQ ID NO:34 | 10.60 | 47 |
| Pyr-SKFKQISI-Nle-WQ | SEQ ID NO:35 | 6.88 | 164 |
| Pyr-SKKKQISI-Nle-WQ | SEQ ID NO:36 | 7.08 | 466 |
| Pyr-SKVKQISI-Nle-WQ | SEQ ID NO:37 | 9.17 | 88 |
| Pyr-SKWKQISI-Nle-WQ | SEQ ID NO:38 | 6.34 | 95 |
| Pyr-SKIRQISI-Nle-WQ | SEQ ID NO:39 | 6.70 | 106 |
| Pyr-SKIKRISI-Nle-WQ | SEQ ID NO:40 | 7.10 | 1184 |
| Pyr-SKIKSISI-Nle-WQ | SEQ ID NO:41 | 6.86 | 70 |
| Pyr-SKIKQIMI-Nle-WQ | SEQ ID NO:42 | 6.69 | 21 |
| Pyr-SKIKQISIFWQ | SEQ ID NO:43 | 9.32 | 37 |
| Pyr-SKIKQISIIWQ | SEQ ID NO:44 | 8.44 | 141 |
| Pyr-SKIKQISILWQ | SEQ ID NO:45 | 8.99 | 102 |
| Pyr-SKIKQISIMWQ | SEQ ID NO:46 | 9.32 | 73 |
| Pyr-SKIKQISIQWQ | SEQ ID NO:47 | 8.52 | 1057 |
| Pyr-SKIKQISITWQ | SEQ ID NO:48 | 7.76 | 1028 |
| Pyr-SKIKQISIWWQ | SEQ ID NO:49 | 8.63 | 41 |
| Pyr-SKIKQISIYWQ | SEQ ID NO:50 | 9.25 | 101 |
| Pyr-SKIKQISI-Nle-FQ | SEQ ID NO:51 | 6.73 | 36 |
| Pyr-SKIKQISI-Nle-WH | SEQ ID NO:52 | 8.96 | 91 |
| Pyr-SKIKQISI-Nle-WK | SEQ ID NO:53 | 6.67 | 1066 |
| Pyr-SKIKQISI-Nle-WS | SEQ ID NO:54 | 8.48 | 44 |
| Ac-KGGQSKIKQISI-Nle-WQ | SEQ ID NO:55 | 9.63 | 708 |
| Ac-KGQSKIKQISI-Nle-WQ | SEQ ID NO:56 | 10.79 | 1206 |
| Ac-KQSKIKQISI-Nle-WQ | SEQ ID NO:57 | 11.16 | 628 |
| Ac-KISKIKQISI-Nle-WQ | SEQ ID NO:58 | 9.08 | 816 |
| Ac-HHQSKIKQISI-Nle-WQ | SEQ ID NO:59 | 9.18 | 895 |
| Pyr-SKIKQISI-Nle-WQHH | SEQ ID NO:60 | 6.88 | 1358 |
| Ac-SKIKQISI-Nle-WQ | SEQ ID NO:61 | 8.76 | 81 |
| Pyr-KIKQISI-Nle-WQ | SEQ ID NO:62 | 10.40 | 39 |
| ASKIKQISI-Nle-WQ | SEQ ID NO:63 | 9.35 | 430 |
| DSKIKQISI-Nle-WQ | SEQ ID NO:64 | 8.53 | 85 |
| ESKIKQISI-Nle-WQ | SEQ ID NO:65 | 6.54 | 136 |
| FSKIKQISI-Nle-WQ | SEQ ID NO:66 | 7.10 | 47 |
| GSKIKQISI-Nle-WQ | SEQ ID NO:67 | 7.72 | 414 |
| ISKIKQISI-Nle-WQ | SEQ ID NO:68 | 8.54 | 559 |
| KSKIKQISI-Nle-WQ | SEQ ID NO:69 | 8.67 | 208 |
| LSKIKQISI-Nle-WQ | SEQ ID NO:70 | 9.37 | 584 |
| MSKIKQISI-Nle-WQ | SEQ ID NO:71 | 9.69 | 531 |
| NSKIKQISI-Nle-WQ | SEQ ID NO:72 | 9.32 | 682 |
| QSKIKQISI-Nle-WQ | SEQ ID NO:73 | 10.45 | 798 |
| SSKIKQISI-Nle-WQ | SEQ ID NO:74 | 10.10 | 672 |
| TSKIKQISI-Nle-WQ | SEQ ID NO:75 | 9.06 | 603 |
| VSKIKQISI-Nle-WQ | SEQ ID NO:76 | 9.44 | 705 |
| WSKIKQISI-Nle-WQ | SEQ ID NO:77 | 7.28 | 54 |
| YSKIKQISI-Nle-WQ | SEQ ID NO:78 | 10.04 | 709 |
| Pyr-Abu-KIKQISI-Nle-WQ | SEQ ID NO:79 | 7.52 | 761 |
| Pyr-AKIKQISI-Nle-WQ | SEQ ID NO:80 | 6.98 | 926 |
| Pyr-SKIKQISI-Nva-WQ | SEQ ID NO:81 | 4.59 | 966 |
| Pyr-SKIKQISI-Gly(C5)-WQ | SEQ ID NO:82 | 4.86 | 44 |
| Pyr-SKIKQISI-Gly(C6)-WQ | SEQ ID NO:83 | 4.54 | 34 |
| Me2Q-SKIKQISI-Nle-WQ | SEQ ID NO:84 | 6.40 | 973 |
| Dpr-bAla-QSKIKQISI-Nle-WQ | SEQ ID NO:85 | 7.49 | 904 |
| Pyr-CKIKQISI-Nle-WQ | SEQ ID NO:86 | 10.28 | 23 |

The above experiment was repeated for various peptides to compare the transduction efficacy to the known transducing peptide PTD-B (SEQ ID NO:88). The results are shown in Table 2 below.

**Table 2: Transduction efficacy of various peptides vs. PTD-B**

| Amino acid sequence | SEQ ID NO: | Transduction efficacy vs. PTD-B (@ 1.25 µg/ml) | Solubility LCMS [µg/mL] |
|---|---|---|---|
| Pyr-SKLKQISI-Nle-WQ | SEQ ID NO:1 | 1.23 | 1143 |
| PSKIKQISI-Nle-WQ | SEQ ID NO:2 | 1.27 | 982 |
| HSKIKQISI-Nle-WQ | SEQ ID NO:5 | 1.33 | 1006 |
| Pyr-Dpr-KIKQISI-Nle-WQ | SEQ ID NO:7 | 1.67 | 1084 |
| Ac-KSKIKQISI-Nle-WQ | SEQ ID NO:10 | 1.40 | 1123 |
| Ac-RSKIKQISI-Nle-WQ | SEQ ID NO:11 | 1.39 | 1151 |
| Ac-Orn-SKIKQISI-Nle-WQ | SEQ ID NO:14 | 1.25 | 1089 |
| Ac-HSKIKQISI-Nle-WQ | SEQ ID NO:23 | 1.58 | 48 |
| Ac-PSKIKQISI-Nle-WQ | SEQ ID NO:25 | 1.38 | 134 |
| Pyr-AKIKQISI-Nle-WQ | SEQ ID NO:29 | 1.32 | 121 |
| Pyr-GKIKQISI-Nle-WQ | SEQ ID NO:30 | 1.34 | 60 |
| Pyr-HKIKQISI-Nle-WQ | SEQ ID NO:31 | 1.49 | 53 |
| Pyr-SKVKQISI-Nle-WQ | SEQ ID NO:37 | 1.25 | 88 |
| Ac-KQSKIKQISI-Nle-WQ | SEQ ID NO:57 | 1.23 | 628 |
| Pyr-KIKQISI-Nle-WQ | SEQ ID NO:62 | 1.23 | 39 |
| ASKIKQISI-Nle-WQ | SEQ ID NO:63 | 1.25 | 430 |
| LSKIKQISI-Nle-WQ | SEQ ID NO:70 | 1.25 | 584 |
| MSKIKQISI-Nle-WQ | SEQ ID NO:71 | 1.22 | 531 |
| QSKIKQISI-Nle-WQ | SEQ ID NO:73 | 1.32 | 798 |
| SSKIKQISI-Nle-WQ | SEQ ID NO:74 | 1.27 | 672 |
| YSKIKQISI-Nle-WQ | SEQ ID NO:78 | 1.22 | 709 |
| Pyr-Abu-KIKQISI-Nle-WQ | SEQ ID NO:79 | 1.38 | 761 |
| Pyr-AKIKQISI-Nle-WQ | SEQ ID NO:80 | 1.28 | 926 |
| Pyr-CKIKQISI-Nle-WQ | SEQ ID NO:86 | 1.32 | 23 |
| Pyr-CKIKQISIMWQ | SEQ ID NO:87 | 1.31 | n.d. |
| Pyr-CKIKQIINMWQ (Reference PTD-B) | SEQ ID NO:88 | 1.00 | 11 |

### Retroviral gene manipulation and preparation CAR T-cells for adoptive T cell transfer

Splenocytes of either naive C57BI/6-Thyl.I+ or Balb/c-Thyl.I+ are isolated and pre-activated by Dynabeads^{™} Mouse T-Activator CD3/CD28 in a bead to T cell ratio of 1:1 (Invitrogen) in the presences of 5 ng/mL recombinant human (rh) IL-7 and 5 ng/mL rh IL-15 (Miltenyi Biotec). For transduction of murine cells, one or more peptides of the invention are prepared as a 10 mg/mL stock solution in DMSO, diluted with PBS to a working solution concentration of 1 mg/mL, added to MLV-E-pseudotyped retroviral supernatants at amounts to obtain a concentration of 6.25 to 50 µg/mL, for example 12.5 µg/mL, and incubated for 5 min at room temperature. 0.5-0.6×10⁶ cells/cm² are inoculated with the virus/peptide mixture (e.g. 10 vol.% virus/peptide mixture and 90 vol.% cells) and incubated under standard conditions. After overnight cultivation/24 h, transduction can be repeated with freshly prepared virus/peptide solution. 72 h after pre-activation, Dynabeads^{™} Mouse T-Activator CD3/CD28 are removed from culture and cells are expanded in the presence of 5 ng/mL rh IL-7 and 5 ng/mL rh IL-15. After Ficoll cleaning, cells are washed twice with PBS to remove serum proteins and are then prepared for adoptive cell transfer (ACT). pES12.6 based retroviral vectors containing CLDN6-CAR-BBz encoded as well as enhanced firefly luciferase (effLuc; Rabinovich et al.BA, PNAS (2008) PNAS 105(38):14342-6) and eGFP (enhanced green fluorescence protein) reporter gene, which express separately using 2A-splice elements (Szymczak et al. AL, Nature Biotechnology, (2004) Nat Biotechnol. 22(5):589-94) are used for transduction.

### Adoptive T-cells transfer of murine T-cells and RNA(LIP) vaccination

Gamma-retroviral transduced CAR congenic Thyl.I+ T-cells are intravenously (i.v.) transferred into total body irradiated (XRAD320) CS BL/SBrdCrHsd-Ty^ or BALB/c donor mice, respectively. Subsequently, mice are intravenously (i.v.) vaccinated with an F12:RIMA ratio of 1.3 : 2 of antigen encoding RNA(LIP) at various time points after ACT. CAR *in vivo* expansion is analyzed via whole body bioluminescence imaging and anti-tumoral efficacy is analyzed by tumor monitoring.

## Claims

1. Peptide comprising the amino acid sequence:
X¹X²X³X⁴X⁵X⁶X⁷X⁸X⁹X¹⁰X¹¹X¹²
wherein
X¹ is absent or is any amino acid, preferably pyroglutamic acid (Pyr), A, D, E, F, G, H, I, K, L, M, N, P, Q, R, S, T, V, W, Y, or ornithine (Orn), more preferably pyroglutamic acid (Pyr), H, K, R, P, T, Q or ornithine (Orn), optionally comprising an N-terminal protecting group, preferably selected from C₁-C₁₀-alkyl-C(=O)-, C₁-C₁₀-hydroxyalkyl-C(=O)-, C₆-C₁₄-aryl-C(=O)-, C₁-C₁₀-alkyl-NH-C(=O)-, C₆-C₁₄-aryl-NH-C(=O)-; (C₁-C₁₀-alkyl)₂N-C(=O)-, (C₆-C₁₄-aryl)₂N-C(=O)-, C₁-C₁₀-alkyl-, (C₁-C₁₀-alkyl)₂-, C₁-C₁₀-alkyl-O-C(=O)-, C₆-C₁₄-aryl-O-C(=O)-, C₁-C₁₀-alkyl-S(O)₂-, C₆-C₁₄-aryl-S(O)₂-, more preferably acetyl, hydroxyacetyl, propionyl, benzoyl, methyl-NH-C(=O), phenyl-NH-C(=O)-, methyl-O-C(=O)-, phenyl-O-C(=O)-, methyl-S(O)₂-, phenyl-S(O)₂-, CH₃, and (CH₃)₂;
X² is absent or S, diaminopropionic acid (Dpr), aminobutyric acid (Abu), H, G, A, P, N, L-homoserine (Hse), or C;
X³ is K or R;
X⁴ is I, L, V, F, W, Y, M, or K;
X⁵ is K or R;
X⁶ is Q, K, I, R, T, or S;
X⁷ is I, L or V;
X⁸ is S, F, A or M;
X⁹ is I or V;
X¹⁰ is L-norleucine (Nle), L-norvaline (Nva), F, Y, M, L, I, Q, T, 2-aminoheptanoic acid, 2-aminooctanoic acid, or W;
X¹¹ is W, F, I, or L;
X¹² is Q, H, R, S or K.

2. The peptide of claim 1, wherein
(1) X¹ is Pyr, H, K, R, P, T, Q or Orn, preferably Pyr, H, P or R;
(2) X² is S, Dpr, Abu, H, G, A, P, N or C, preferably S, Dpr, H, G, A, or C;
(3) X³ is K or R;
(4) X⁴ is I, L or V, preferably I;
(5) X⁵ is K;
(6) X⁶ is Q or K, preferably Q;
(7) X⁷ is I;
(8) X⁸ is S or F, preferably S;
(9) X⁹ is I;
(10) X¹⁰ is Nle or F, preferably Nle;
(11) X¹¹ is W; and/or
(12) X¹² is Q, H, R or S, preferably Q, R or H.

3. The peptide of claim 1 or 2, wherein the peptide comprises the amino acid sequence:
(1)
X¹X²X³X⁴KX⁶IX⁸IX¹⁰WX¹²;
(2)
X¹X²X³IKX⁶IX⁸IX¹⁰WX¹²;
(3)
X¹X²X³LKX⁶IX⁸IX¹⁰WX¹²;
(4)
X¹X²X³VKX⁶IX⁸IX¹⁰WX¹²;
(5)
X¹X²X³X⁴KQIX⁸IX¹⁰WX¹²;
(6)
X¹X²X³X⁴KKIX⁸IX¹⁰WX¹²;
(7)
X¹X²X³X⁴KIIX⁸IX¹⁰WX¹²
(8)
X¹X²X³X⁴KX⁶ISIX¹⁰WX¹²;
(9)
X¹X²X³X⁴KX⁶IFIX¹⁰WX¹²;
(10)
X¹X²X³X⁴KX⁶IX⁸I-Nle-WX¹²;
(11)
X¹X²X³X⁴KX⁶IX⁸IX¹⁰WQ;
(12)
X¹X²X³X⁴KX⁶IX⁸IX¹⁰WR;
(13)
X¹X²X³IKQISIX¹⁰X¹¹X¹²; or
(14)
X¹X²X³IKQISIX¹⁰WX¹².

4. The peptide of any one of claims 1 to 3, wherein the peptide comprises the amino acid sequence:
(1)
X¹X²KX⁴KX⁶IX⁸IX¹⁰WX¹²;
(2)
X¹X²KIKX⁶IX⁸IX¹⁰WX¹²;
(3)
X¹X²KLKX⁶IX⁸IX¹⁰WX¹²;
(4)
X¹X²KVKX⁶IX⁸IX¹⁰WX¹²;
(5)
X¹X²KX⁴KQIX⁸IX¹⁰WX¹²;
(6)
X¹X²KX⁴KKIX⁸IX¹⁰WX¹²;
(7)
X¹X²KX⁴KIIX⁸IX¹⁰WX¹²
(8)
X¹X²KX⁴KX⁶ISIX¹⁰WX¹²;
(9)
X¹X²KX⁴KX⁶IFIX¹⁰WX¹²;
(10)
X¹X²KX⁴KX⁶IX⁸I-Nle-WX¹²;
(11)
X¹X²KX⁴KX⁶IX⁸IX¹⁰WQ;
(12)
X¹X²KX⁴KX⁶IX⁸IX¹⁰WR;
(13)
X¹X²KX⁴KX⁶IX⁸IX¹⁰WH;
(14)
X¹X²KIKQISIX¹⁰X¹¹X¹²; or
(15)
X¹X²KIKQISIX¹⁰WX¹².

5. The peptide of any one of claims 1 to 4, wherein the peptide comprises the amino acid sequence:
X¹X²KIKQISIX¹⁰WX¹²
wherein X¹⁰ is preferably Nle and X¹² is preferably Q.

6. The peptide of any one of claims 1 to 5, wherein the peptide comprises or consists of the amino acid sequence
(1) Pyr-SKLKQISI-Nle-WQ (SEQ ID NO:1);
(2) PSKIKQISI-Nle-WQ (SEQ ID NO:2);
(3) HHKIKQISI-Nle-WQ (SEQ ID NO:3);
(4) RSRIKQISI-Nle-WQ (SEQ ID NO:4), wherein the N-terminus is acetylated;
(5) HSKIKQISI-Nle-WQ (SEQ ID NO:5);
(6) Pyr-Hse-KIKQISI-Nle-WQ (SEQ ID NO:6);
(7) Pyr-Dpr-KIKQISI-Nle-WQ (SEQ ID NO:7);
(8) H-Dpr-KIKQISI-Nle-WQ (SEQ ID NO:8);
(9) R-Dpr-KIKQISI-Nle-WQ (SEQ ID NO:9);
(10) KSKIKQISI-Nle-WQ (SEQ ID NO:10), wherein the N-terminus is acetylated;
(11) RSKIKQISI-Nle-WQ (SEQ ID NO:11), wherein the N-terminus is acetylated;
(12) Pyr-SKIKKISI-Nle-WQ (SEQ ID NO:12);
(13) Pyr-SKIKQISI-Nle-WR (SEQ ID NO:13);
(14) Orn-SKIKQISI-Nle-WQ (SEQ ID NO:14), wherein the N-terminus is acetylated;
(15) RSKIKQISI-Nle-WQ (SEQ ID NO:15);
(16) HCKIKQISI-Nle-WQ (SEQ ID NO:16);
(17) RHKIKQISI-Nle-WQ (SEQ ID NO:17);
(18) RCKIKQISI-Nle-WQ (SEQ ID NO:18);
(19) RSRIKQISI-Nle-WQ (SEQ ID NO:19);
(20) Pyr-SKIKQISI-Nle-WQ (SEQ ID NO:20);
(21) ASKIKQISI-Nle-WQ (SEQ ID NO:21), wherein the N-terminus is acetylated;
(22) GSKIKQISI-Nle-WQ (SEQ ID NO:22), wherein the N-terminus is acetylated;
(23) HSKIKQISI-Nle-WQ (SEQ ID NO:23), wherein the N-terminus is acetylated;
(24) NSKIKQISI-Nle-WQ (SEQ ID NO:24), wherein the N-terminus is acetylated;
(25) PSKIKQISI-Nle-WQ (SEQ ID NO:25), wherein the N-terminus is acetylated;
(26) QSKIKQISI-Nle-WQ (SEQ ID NO:26), wherein the N-terminus is acetylated;
(27) SSKIKQISI-Nle-WQ (SEQ ID NO:27), wherein the N-terminus is acetylated;
(28) TSKIKQISI-Nle-WQ (SEQ ID NO:28), wherein the N-terminus is acetylated;
(29) Pyr-AKIKQISI-Nle-WQ (SEQ ID NO:29);
(30) Pyr-GKIKQISI-Nle-WQ (SEQ ID NO:30);
(31) Pyr-HKIKQISI-Nle-WQ (SEQ ID NO:31);
(32) Pyr-NKIKQISI-Nle-WQ (SEQ ID NO:32);
(33) Pyr-PKIKQISI-Nle-WQ (SEQ ID NO:33);
(34) Pyr-SRIKQISI-Nle-WQ (SEQ ID NO:34);
(35) Pyr-SKFKQISI-Nle-WQ (SEQ ID NO:35);
(36) Pyr-SKKKQISI-Nle-WQ (SEQ ID NO:36);
(37) Pyr-SKVKQISI-Nle-WQ (SEQ ID NO:37);
(38) Pyr-SKWKQISI-Nle-WQ (SEQ ID NO:38);
(39) Pyr-SKIRQISI-Nle-WQ (SEQ ID NO:39);
(40) Pyr-SKIKRISI-Nle-WQ (SEQ ID NO:40);
(41) Pyr-SKIKSISI-Nle-WQ (SEQ ID NO:41);
(42) Pyr-SKIKQIMI-Nle-WQ (SEQ ID NO:42);
(43) Pyr-SKIKQISIFWQ (SEQ ID NO:43);
(44) Pyr-SKIKQISIIWQ (SEQ ID NO:44);
(45) Pyr-SKIKQISILWQ (SEQ ID NO:45);
(46) Pyr-SKIKQISIMWQ (SEQ ID NO:46);
(47) Pyr-SKIKQISIQWQ (SEQ ID NO:47);
(48) Pyr-SKIKQISITWQ (SEQ ID NO:48);
(49) Pyr-SKIKQISIWWQ (SEQ ID NO:49);
(50) Pyr-SKIKQISIYWQ (SEQ ID NO:50);
(51) Pyr-SKIKQISI-Nle-FQ (SEQ ID NO:51);
(52) Pyr-SKIKQISI-Nle-WH (SEQ ID NO:52);
(53) Pyr-SKIKQISI-Nle-WK (SEQ ID NO:53);
(54) Pyr-SKIKQISI-Nle-WS (SEQ ID NO:54);
(55) KGGQSKIKQISI-Nle-WQ (SEQ ID NO:55), wherein the N-terminus is acetylated;
(56) KGQSKIKQISI-Nle-WQ (SEQ ID NO:56), wherein the N-terminus is acetylated;
(57) KQSKIKQISI-Nle-WQ (SEQ ID NO:57), wherein the N-terminus is acetylated;
(58) KISKIKQISI-Nle-WQ (SEQ ID NO:58), wherein the N-terminus is acetylated;
(59) HHQSKIKQISI-Nle-WQ (SEQ ID NO:59), wherein the N-terminus is acetylated;
(60) Pyr-SKIKQISI-Nle-WQHH (SEQ ID NO:60);
(61) SKIKQISI-Nle-WQ (SEQ ID NO:61), wherein the N-terminus is acetylated;
(62) Pyr-KIKQISI-Nle-WQ (SEQ ID NO:62);
(63) ASKIKQISI-Nle-WQ (SEQ ID NO:63);
(64) DSKIKQISI-Nle-WQ (SEQ ID NO:64);
(65) ESKIKQISI-Nle-WQ (SEQ ID NO:65);
(66) FSKIKQISI-Nle-WQ (SEQ ID NO:66);
(67) GSKIKQISI-Nle-WQ (SEQ ID NO:67);
(68) ISKIKQISI-Nle-WQ (SEQ ID NO:68);
(69) KSKIKQISI-Nle-WQ (SEQ ID NO:69);
(70) LSKIKQISI-Nle-WQ (SEQ ID NO:70);
(71) MSKIKQISI-Nle-WQ (SEQ ID NO:71);
(72) NSKIKQISI-Nle-WQ (SEQ ID NO:72);
(73) QSKIKQISI-Nle-WQ (SEQ ID NO:73);
(74) SSKIKQISI-Nle-WQ (SEQ ID NO:74);
(75) TSKIKQISI-Nle-WQ (SEQ ID NO:75);
(76) VSKIKQISI-Nle-WQ (SEQ ID NO:76);
(77) WSKIKQISI-Nle-WQ (SEQ ID NO:77);
(78) YSKIKQISI-Nle-WQ (SEQ ID NO:78);
(79) Pyr-Abu-KIKQISI-Nle-WQ (SEQ ID NO:79);
(80) Pyr-AKIKQISI-Nle-WQ (SEQ ID NO:80);
(81) Pyr-SKIKQISI-Nva-WQ (SEQ ID NO:81);
(82) Pyr-SKIKQISI-Gly(C5)-WQ (SEQ ID NO:82);
(83) Pyr-SKIKQISI-Gly(C6)-WQ (SEQ ID NO:83);
(84) Me2Q-SKIKQISI-Nle-WQ (SEQ ID NO:84);
(85) Dpr-bAla-QSKIKQISI-Nle-WQ (SEQ ID NO:85); or
(86) Pyr-CKIKQISI-Nle-WQ (SEQ ID NO:86).

7. The peptide of claim 6, wherein the peptide comprises or consists of the amino acid sequence Pyr-SKLKQISI-Nle-WQ (SEQ ID NO:1);
PSKIKQISI-Nle-WQ (SEQ ID NO:2);
HHKIKQISI-Nle-WQ (SEQ ID NO:3);
RSRIKQISI-Nle-WQ (SEQ ID NO:4), wherein the N-terminus is acetylated;
KSKIKQISI-Nle-WQ (SEQ ID NO:10), wherein the N-terminus is acetylated;
RSKIKQISI-Nle-WQ (SEQ ID NO:11), wherein the N-terminus is acetylated;
Pyr-SKIKKISI-Nle-WQ (SEQ ID NO:12);
Pyr-SKIKQISI-Nle-WR (SEQ ID NO:13);
Orn-SKIKQISI-Nle-WQ (SEQ ID NO:14), wherein the N-terminus is acetylated;
HSKIKQISI-Nle-WQ (SEQ ID NO:5);
RSKIKQISI-Nle-WQ (SEQ ID NO:15);
Pyr-Hse-KIKQISI-Nle-WQ (SEQ ID NO:6);
Pyr-Dpr-KIKQISI-Nle-WQ (SEQ ID NO:7);
H-Dpr-KIKQISI-Nle-WQ (SEQ ID NO:8);
HCKIKQISI-Nle-WQ (SEQ ID NO:16);
RHKIKQISI-Nle-WQ (SEQ ID NO:17);
R-Dpr-KIKQISI-Nle-WQ (SEQ ID NO:9);
RCKIKQISI-Nle-WQ (SEQ ID NO:18);
RSRIKQISI-Nle-WQ (SEQ ID NO:19);
HSKIKQISI-Nle-WQ (SEQ NO:23), wherein the N-terminus is acetylated;
PSKIKQISI-Nle-WQ (SEQ NO:25), wherein the N-terminus is acetylated;
Pyr-AKIKQISI-Nle-WQ (SEQ NO:29);
Pyr-GKIKQISI-Nle-WQ (SEQ NO:30);
Pyr-HKIKQISI-Nle-WQ (SEQ NO:31);
Pyr-SKVKQISI-Nle-WQ (SEQ NO:37);
KQSKIKQISI-Nle-WQ (SEQ NO:57), wherein the N-terminus is acetylated;
Pyr-KIKQISI-Nle-WQ (SEQ NO:62);
ASKIKQISI-Nle-WQ (SEQ NO:63);
LSKIKQISI-Nle-WQ (SEQ NO:70);
MSKIKQISI-Nle-WQ (SEQ NO:71);
QSKIKQISI-Nle-WQ (SEQ NO:73);
SSKIKQISI-Nle-WQ (SEQ NO:74);
YSKIKQISI-Nle-WQ (SEQ NO:78);
Pyr-Abu-KIKQISI-Nle-WQ (SEQ NO:79);
Pyr-AKIKQISI-Nle-WQ (SEQ NO:80); or
Pyr-CKIKQISI-Nle-WQ (SEQ NO:86);
preferably any one of
Pyr-SKLKQISI-Nle-WQ (SEQ ID NO:1);
PSKIKQISI-Nle-WQ (SEQ ID NO:2);
HHKIKQISI-Nle-WQ (SEQ ID NO:3); and
RSRIKQISI-Nle-WQ (SEQ ID NO:4), wherein the N-terminus is acetylated.

8. The peptide of any one of claims 1 to 7, wherein
(1) the peptide is 10 to 30 amino acids in length, preferably 10 to 20 amino acids in length, more preferably 11 to 15 amino acids in length; and/or
(2) the peptide has the ability to improve the transduction efficiency of a virus or viral vector.

9. Composition comprising at least one peptide of any one of claims 1 to 8, optionally further comprising at least one virus or viral vector.

10. The peptides of claims 1 to 8 or the composition of claim 9 for use
(1) in transducing a eukaryotic cell; and/or
(2) as a medicament or biopharmaceutical; and/or
(3) in gene and cell therapy.

11. Method for the infection of a eukaryotic cell by a virus or viral vector, comprising contacting the cell with the virus or viral vector in the presence of at least one peptide according to any one of claims 1 to 8.

12. Eukaryotic cell transduced with a composition of claim 9 or obtained by the method of claim 11.

13. Method for increasing the efficiency of a nucleic acid transfer into a eukaryotic target cell with a viral vector, comprising contacting the target cell with the viral vector in the presence of at least one peptide according to any one of claims 1 to 8.

14. Use of at least one peptide of any one of claims 1 to 8
(1) for promoting the infection of a eukaryotic cell by a virus or viral vector; or
(2) in a method of diagnosis, for example for diagnosing a disease or disorder.

15. The peptides or composition for use according to claim 10, the method of claim 11 or 13, the eukaryotic cell of claim 14 or the use of claim 14, wherein the eukaryotic cell is selected from immune cells, preferably lymphocytes and/or the virus is selected from retroviruses.
